# EUROPEAN PATENT APPLICATION

(11) **EP 1 712 125 A1**
(43) Date of publication of application: **18.10.2006**
(21) Application number: 04820285.7
(22) Date of filing: 09.12.2004
(51) Int. Cl.: A01K 67/027, C12N 15/09, G01N 33/50

(54) **DISEASE MODEL ANIMAL EXPRESSING MEGSIN/RAGE/iNOS AND METHOD OF EVALUATING COMPOUND WITH THE USE OF THE ANIMAL**

(30) Priority: 12.12.2003 JP 2003415779
(71) Applicant: Japan Science and Technology Agency, Kawaguchi-shi, Saitama 332-0012 (JP); Gene Thesis Co., Ltd., Kanagawa 230-0046 (JP)
(72) Inventor: MIYATA, Toshio, Isehara-shi, Kanagawa 2591132 (JP); KUROKAWA, Kiyoshi 401 Ichigayahills, Tokyo 1620061 (JP); YAMAMOTO, Hiroshi, Kanazawa-shi, Ishikawa 9218105 (JP); OKAMOTO, Hiroshi, Sendai-shi, Miyagi 9893201 (JP)
(74) Representative: Grünecker, Kinkeldey, Stockmair & Schwanhäusser Anwaltssozietät
(86) International application number: PCT/JP2004/018413
(87) International publication number: WO 2005/055706

(57) **Abstract**

Triple Tg (megsin/RAGE/iNOS-Tg) was created by crossing megsin-Tg with RAGE/iNOS-Tg. The megsin/RAGE/iNOS-Tg develops marked pathologies of diabetic nephropathy unfound in conventional models at early stages, and various pathological conditions such as glomerular hypertrophy were observed uniformly in the megsin/RAGE/iNOS-Tg mice. In addition, it was also found that animals exhibiting these symptoms were useful as a disease model animal for diabetic nephropathy. Specifically, the disease model animals of the present invention strongly express the megsin gene, a gene encoding the receptor for advanced glycation end-products, and an inducible nitric oxide synthase gene. As a result, accompanying kidney function disorders of glomerular failure develop at early stages.

## Description

### Technical Field

The present invention relates to disease model animals, in particular, model animals for accompanying kidney function disorders of hyperglycemia, such as diabetic nephropathy, and accompanying kidney function disorders of glomerular disorders. The present invention also relates to methods for evaluating the therapeutic effect of compounds on kidney function disorders using the disease model animals.

### Background Art

In recent years, the number of diabetes patients has steadily increased as the lifestyle and diet become Westernized. Diabetes mellitus is a disease in which persistent hyperglycemia causes various tissue disorders, namely complications, throughout the whole body. In addition to the so-called great vessel complications, such as coronary artery sclerosis and cerebrovascular disorders, the diabetes mellitus-characteristic pathological conditions arise: retinopathy, neuropathy, and nephropathy, which together are called "three major complications". In particular, nephropathy is a fatal complication in diabetes patients and a major cause of end-stage renal disease (ESRD) in advanced countries (Non-Patent Document 1).

Since approximately 30% of insulin-dependent diabetes mellitus (IDDM) patients develop diabetic nephropathy and need dialysis or kidney transplantation and such, and eventually their quality of life (QOL) is severely impaired (Non-Patent Documents 1 and 2). Furthermore, average life spans of both male and female diabetes patients are shortened by 10 or more years compared with the general population. Persistent proteinuria, progressive impairment of kidney function, and histopathological mesangial cell proliferation are characteristic symptoms of diabetic nephropathy.

Thus, elucidation of molecular mechanisms for the onset and progression of diabetes mellitus complications and discovery of preventive and therapeutic methods are thought to be important objectives medically and socially.

Model animals are needed for elucidating the onset mechanism of diabetic nephropathy and for developing therapeutic and preventive methods. Various studies are being conducted to create such model animals. However, there are no known animal models that exhibit changes such as those observed in human diabetic nephropathy (Non-Patent Document 3), except only a few models such as diabetes mellitus animal models, a spontaneous non-obese diabetic (NOD) mouse which develops limited disorders such as mild mesangial cell sclerosis (Non-Patent Document 4), and rodent model animals with chemically induced diabetes mellitus (Non-Patent Document 5). Furthermore, none of these known animal models are sufficient in terms of reproducibility and pathological findings. Specifically, mild proliferation of mesangial matrix and resulting glomerular hypertrophy are pathological findings also found in conventional diabetic nephropathy models. However, there are no known animal models that have all phenotypes (biochemical and pathological findings) which resemble those of human diabetic nephropathy. Furthermore, there is no model that exhibits nodular glomerulosclerosis which is observed in pathological conditions of an advanced stage. Thus, the study of diabetic nephropathy becomes difficult. Under the circumstances described above, it is important to create an animal model with phenotypes that resemble those of human diabetic nephropathy in order to understand the pathophysiology of diabetic nephropathy.

As described above, diabetic nephropathy is one of the complications of diabetes mellitus, and the following *in vivo* reaction is thought to be involved in its onset mechanism.

When proteins are exposed to reducing sugars such as glucose, a non-enzymatic glycation reaction is induced, and glycation products such as reversible Schiff base and Amadori rearrangement compound are formed (Non-Patent Document 6). This process is called "early reaction". Then, irreversibly advanced glycation end-products (AGE) are formed *via* complicated intramolecular rearrangement reactions, such as condensation, cleavage, and cross-linking (Non-Patent Document 6). This series of reactions is called "glycation". AGE is a generic name for such structures formed *via* the process described above. In diabetes mellitus, the long-lasting hyperglycemic state accelerates the reaction, and AGE accumulates in the circulating blood and various tissues (Non-Patent Document 6). The formation of AGE is considered to be one of the causes for diabetic complications.

Meanwhile, attention has been drawn to the mechanism which employs a cellular response induced by the cell surface receptor recognizing AGE as a specific ligand (receptor for advanced glycation end-products: RAGE; hereinafter referred to as "RAGE").

RAGE is a membrane protein with a molecular weight of about 35 kDa and belongs to the immunoglobulin superfamily isolated and identified from bovine lung in 1992. RAGE is a cell surface receptor recognizing AGE (Non-Patent Documents 7 and 8). Recently, the full length glycosylated human RAGE was found to be 55 kDa (Non-Patent Document 9). RAGE is a single-transmembrane receptor having three immunoglobulin-like extracellular domains, and has a short intracellular domain that is estimated to be essential for signal transduction. The AGE ligand-binding domain of RAGE has been found to locate within the most N-terminal of the three immunoglobulin-like domains. In addition, RAGE has been found to be expressed in many types of cells, including macrophages and cells constituting blood vessel, such as vascular endothelial cell, smooth muscle cell, pericyte, and kidney mesangial cell (Non-Patent Documents 7 and 8). It has also been revealed that in endothelial cells, one of the cells listed above, the AGE ligand itself induces the expression of RAGE gene at the transcriptional level (Non-Patent Document 10).

To examine the *in vivo* impact of the AGE-RAGE system on vascular complications of diabetes mellitus, in particular, nephropathy and retinopathy, the present inventors previously created and analyzed a transgenic mouse (RAGE-Tg) whose vascular cells overexpress RAGE (Non-Patent Document 11). Specifically, the RAGE-Tg was created using a transgene in which the human RAGE gene had been ligated downstream of a vascular endothelial cell-specific mouse flk-1 promotor. Then, to induce diabetes mellitus, the mouse was crossed with a transgenic (Tg) mouse of a different strain, in which an inducible nitric oxide synthase (iNOS; hereinafter referred to as "iNOS") is overexpressed in a pancreatic β cell-specific manner (Non-Patent Document 12), and a double-Tg (RAGE/iNOS-Tg) mouse which develops insulin-dependent diabetes mellitus at early postnatal stages was created.

This RAGE/iNOS-Tg developed kidney failure and progressive glomerulosclerosis similar to that found in human diabetic nephropathy. Specifically, when the degree of nephropathy progression was compared between RAGE-Tg littermates with diabetes mellitus and non-Tg male mice with diabetes mellitus (control), the kidney disorder had been significantly exacerbated in RAGE/iNOS-Tg, while there was no significant difference between the two groups in terms of indices such as blood glucose level, HbAic level, and blood AGE level. That is, the ratio between urine albumin and creatinine, an indicator of proteinuria, was increased in RAGE/iNOS-Tg; the serum creatinine level and kidney-to-body weight ratio were also increased; and histologically significant glomerular hypertrophy and glomerulosclerosis were observed.

Thus, it was proven for the first time that RAGE overexpression could enhance the onset and progression of diabetic nephropathy at the individual level. RAGE was identified as a nephropathy-susceptible gene and it was confirmed that RAGE/iNOS-Tg could serve as the first animal model for understanding the ESRD-mediated process of diabetogenic kidney pathology. Thus, the AGE-RAGE system may be a promising target for preventive and therapeutic methods of diabetic complications.

RAGE/iNOS-Tg is thus an excellent animal model for diabetic complications. However, if an animal model that develops a much more marked kidney function disorder and requires a shorter time period for the onset can be obtained, the model would be more helpful for understanding the pathophysiology of diabetic complications such as diabetic nephropathy, and highly useful for developing therapeutic agents or the like for diabetic complications.

Meanwhile, the present inventors isolated megsin, a gene specifically expressed in mesangial cells (Patent Document 1), and created transgenic mice (megsin-Tg) introduced with megsin. Significant cell proliferation, mainly mesangial cell proliferation, increased mesangial matrix, and deposition of immune complexes consisting of complement and immunoglobulin were seen, and pathological findings of mesangial proliferation such as segmental sclerosis, were found in the glomerular tissues of about 35- to 40-week-old megsin-Tg (Non-Patent Document 13). Based on the findings described above, one could argue that megsin-Tg is useful as an animal model for mesangial proliferative glomerulonephritis. However, megsin-Tg's kidney function was normal and urine proteins or such were not detected. In addition, it takes as long as 35 to 40 weeks after birth for mesangial proliferative glomerulonephritis to develop. Thus, megsin-Tg does not satisfy the above-described requisites for an animal model of diabetic complications.
Patent Document 1: International Publication Number WO 99/15652
Non-Patent Document 1: Bojestig, M., et al., N. Engl. J. Med., 330:15-18 (1994)
Non-Patent Document 2: Krolewski, M., et al., Kidney Int., 50:2041-2046 (1996)
Non-Patent Document 3: Velasquez, M. T., et al., FASEB J., 4:2850-2859 (1990)
Non-Patent Document 4: Doi, T., et al., Lab. Invest., 63:204-212 (1990)
Non-Patent Document 5: Williamson, J. R., et al., Diabetes., 36:813-821 (1987)
Non-Patent Document 6: Brownlee, M., et al., N. Engl. J. Med., 318:1315-1321 (1988)
Non-Patent Document 7: Schmidt, A. M., et al., J. Biol. Chem., 267:14987-14997 (1992)
Non-Patent Document 8: Neeper, M., et al., J. Biol. Chem., 267:14998-15004 (1992)
Non-Patent Document 9: Yonekura, H., et al., Biochem. J., 370:1097-1109 (2003)
Non-Patent Document 10: Tanaka, N., et al., J. Biol. Chem., 275:25781-25790 (2000)
Non-Patent Document 11: Yamamoto, Y., et al., J. Clin. Invest., 108:261-268 (2001)
Non-Patent Document 12: Takamura, T., et al., J. Biol. Chem., 273:2493-2496 (1998)
Non-Patent Document 13: Miyata, T., et ai., J. Clin. Invest., 109: 585-593 (2002)

### Disclosure of the Invention

An objective of the present invention is to provide more useful disease animal models than conventional ones for diabetic nephropathy. Another objective of the present invention is to provide methods for evaluating compounds useful for the treatment of diabetic nephropathy.

The present inventors discovered from their studies that mesangial proliferative glomerulonephritis can be induced by forcedly expressing megsin in glomerulus and RAGE/iNOS-Tg can be an excellent animal model of diabetic complications. Based on these findings, the present inventors further performed dedicated research on the onset mechanisms of kidney function disorders, especially the mechanism of diabetic nephropathy. From this study, a finding leading to the present invention was obtained.

First, triple-Tg (megsin/RAGE/iNOS-Tg) was created by crossing megsin-Tg with RAGE/iNOS-Tg. It was found that this megsin/R.AGE/iNOS-Tg developed diabetic kidney pathology (nodular glomerulosclerosis), which is not found in conventional models, and the onset occurred at an early stage, and kidney pathologies such as glomerular hypertrophy were observed uniformly.

More specifically, in the created triple-Tg mouse, glomerular hypertrophy and cell proliferation in the glomerulus were detected at an early stage, for example, 8 weeks or 16 weeks, and associatively mesangial matrix expansion accelerated. The morphological glomerular abnormalities at early stages result in glomerular disorders (mesangial cell activation), severe tubulointerstitial disorder (fibrosis), interstitial infiltration of macrophages, increased oxidative stress, and such. The triple-Tg mouse exhibits pathological conditions of late-stage diabetic nephropathy (nodular lesion, and adhesion of glomerular tuft and Bowman's capsule) at an extremely early stage of 16 weeks.

Thus, all phenotypes (biochemical and pathological findings) of the triple-Tg are similar to those found in human diabetic nephropathy. In particular, there is no model to date that efficiently exhibits at an early stage the nodular glomerulosclerosis which is observed in the pathological images of a more advanced stage. Thus, the animal model of the present invention is useful as a disease animal model for diabetic nephropathy.

In addition, since the models of the present invention is accelerated by mesangial cell disorder (megsin overexpression), the models have features that allow a more detailed comparison and analysis of the differences in the megsin pathophysiology and mesangial cell pathophysiology in diabetic nephropathy over a wide range of progressional stages of the pathology. Thus, the model animals of the present invention are useful as an evaluation model for developing novel diagnostic methods and drugs that suppress the progression of kidney disorder before late-stage pathologies occur.

The present invention is achieved based on the findings described above, and specifically described below.
[1] A disease model animal expressing megsin gene, a gene encoding the receptor for advanced glycation end-products, and an inducible nitric oxide synthase gene, wherein the model animal comprises a nonhuman mammal.
[2] The disease model animal of [1] introduced with megsin gene, a gene encoding the receptor for advanced glycation end-products, and an inducible nitric oxide synthase gene.
[3] The disease model animal of [1] or [2], which exhibits at least one phenotype selected from the following phenotypes (a) to (f):
   (a) increase in kidney-to-body weight ratio;
   (b) increase in urine albumin level;
   (c) increase in blood triglyceride level;
   (d) underweight (hypogenesis);
   (e) hyperglycemia; and
   (f) hypoinsulinemia.
[4] The disease model animal of any one of [1] to [3], which exhibits in mesangial matrix at least one of the following phenotypes:
   (a) expansion of mesangial matrix;
   (b) enhancement of immunoglobulin and/or complement accumulation; and
   (c) increases of collagen, laminin, and/or fibronectin.
[5] The disease model animal of [1] or [2], which exhibits in tubular interstitium the phenotypes of:
   (a) fibrosis; and/or
   (b) infiltration of inflammatory cells.
[6] The disease model animal of any one of [1] to [5], wherein the megsin gene, the gene encoding the receptor for advanced glycation end-products, and the inducible nitric oxide synthase gene are derived from human.
[7] The disease model animal of any one of [1] to [6], wherein the disease is diabetic nephropathy.
[8] A method for creating a disease model animal, comprising the step of introducing megsin gene, a gene encoding the receptor for advanced glycation end-products, and an inducible nitric oxide synthase gene into a fertilized egg of a nonhuman mammal, wherein the disease model animal comprises a nonhuman mammal in which expressions of the megsin gene, the gene encoding the receptor for advanced glycation end-products, and the inducible nitric oxide synthase gene have been expressed.
[9] A method for evaluating the therapeutic effect of a test compound on kidney function disorder, which comprises the steps of:
   (1) administering a test compound to the disease model animal of any one of claims 1 to 7; and
   (2) detecting the relieving effect on the kidney function disorder of the disease model animal administered with the test compound.
[10] A method for evaluating the therapeutic effect of a test compound on kidney function disorder, which comprises the steps of:
   (1) administering a test compound to the disease model animal of any one of claims 1 to 7; and
   (2) measuring at least any one of kidney-to-body weight ratio, urine albumin level, blood triglyceride level, and urine 8-OHdG level in the disease model animal after administration of the test compound.
[11] A method for evaluating the therapeutic effect of a test compound on kidney function disorder, which comprises the steps of:
   (1) administering a test compound to the disease model animal of any one of [1] to [7]; and
   (2) determining whether the mesangial matrix of the disease model animal is altered or whether the alteration is reduced after administration of the test compound.
[12] The method of [11], wherein the alteration of the mesangial matrix is at least one of:
   (a) expansion of mesangial matrix;
   (b) enhancement of immunoglobulin and/or complement accumulation; and
   (c) increases of collagen, laminin, and/or fibronectin.
[13] A method for evaluating the therapeutic effect of a test compound on kidney function disorder, which comprises the steps of:
   (1) administering a test compound to the disease model animal of any one of [1] to [7]; and
   (2) determining whether the tubular interstitium of the disease model animal is altered or whether the alteration is reduced after administration of the test compound.
[14] The method of [13], wherein the alteration of the tubular interstitium is:
   (a) fibrosis; and/or
   (b) infiltration of inflammatory cells.
[15] The method of any one of [9] to [14], wherein the kidney function disorder is a kidney function disorder that accompanies hyperglycemia.
[16] A method evaluating the therapeutic effect of a test compound on hyperglycemia, which comprises the steps of:
   (1) administering a test compound to the disease model animal of any one of claims 1 to 7; and
   (2) determining the glucose or/insulin level in the disease model animal after administration of the test compound.

The present invention provides disease model animals introduced with megsin, RAGE, and iNOS genes. A marked glomerular disorder (nodular sclerotic lesion) is observed in the model animals of the present invention. This finding supports that the pathological state of the disease model animals of the present invention more closely resembles human diabetic nephropathy. In addition, the model animals of the present invention develop the disease at an early stage (16 weeks old) as compared with conventional model mice (40 weeks old), and various pathological conditions including glomerular hypertrophy are observed to be uniform. Thus, the model animals of the present invention are expected to be highly useful for elucidation of the cause of diabetic nephropathy. The model animals of the present invention are useful not only for analyzing the onset mechanism and pathological state of diabetic nephropathy, but also for the development and screening of therapeutic agents for diabetic nephropathy, medicinal agents, and other purposes.

The present invention also provides disease model animals with symptoms of hyperglycemia. While model animals with hyperglycemia are known, no animal is known to develop hyperglycemia for certain in a short period such as the megsin/RAGE/iNOS-expressing disease model animals of the present invention described above. The disease model animals of the present invention are useful for analyzing and exploring therapeutic methods for various pathological states caused by hyperglycemia, and finding therapeutic methods for hyperglycemia. It has been revealed that hyperglycemia causes various disorders. Thus, the model animals, where various morbid alterations resulting from hyperglycemia can be observed, are useful for analyzing such pathological states.

The disease model animals of the present invention expressing megsin/RAGE/iNOS can be created using transgenic animals. Transgenic animals, from which highly uniform model animals can be provided on a large scale, enable highly accurate experiments. Further, in the disease model animals of the present invention, nodular sclerotic lesions and diabetic nephropathy that closely resembles the human pathological state were observed. As described above, it is of great significance to provide model animals that are true to the actual pathological state.

### Brief Description of the Drawings

Fig. 1 shows the results of confirming the presence of three introduced genes (RAGE, megsin, and iNOS genes) in triple-Tg mice by PCR.
Fig. 2 is a set of photomicrographs showing the results of PAS or PAM staining of the renal glomerular tissues of megsin/RAGE/iNOS-Tg mice and control mice described in Example 2. Top: megsin/RAGE/iNOS-Tg mouse; middle: RAGE/iNOS-Tg mouse; bottom: megsin-Tg mouse. Kidney tissues were collected from mice that were all 16 weeks old.
Fig. 3 is a set of fluorescence photomicrographs showing the results of detecting the accumulation of immunoglobulins in mesangial matrix using the fluorescent antibody method. Left: derived from a control RAGE/iNOS-Tg mouse; right: derived from a megsin/RAGE/iNOS-Tg mouse of the Examples.
Fig. 4 is a set of fluorescence photomicrographs showing the results of detecting the accumulation of complements in mesangial matrix using the fluorescent antibody method. Left: derived from a control RAGE/iNOS-Tg mouse; right: derived from a megsin/RAGE/iNOS-Tg mouse of the Examples.
Fig. 5 is a set of photomicrographs showing the results of staining the type IV collagen present in mesangial matrix by immunohistological staining using an anti-type IV collagen antibody. Left: derived from a megsin/RAGE/iNOS-Tg mouse of the Examples; right, derived from a control megsin-Tg mouse.
Fig. 6 is a set of photomicrographs showing the results of staining the fibronectin present in mesangial matrix by immunohistological staining using an anti-fibronectin antibody. Left: derived from a megsin/RAGE/iNOS-Tg mouse of the Examples; right: derived from a control megsin-Tg mouse.
Fig. 7 is a set of photomicrographs showing the results of staining the laminin present in mesangial matrix by immunohistological staining using an anti-laminin antibody. Left: derived from a megsin/RAGE/iNOS-Tg mouse of the Examples; right: derived from a control megsin-Tg mouse.
Fig. 8 is a set of electron micrographs showing the results of the pathological analysis of megsin/RAGE/iNOS-Tg mouse kidney tissues. Kidney tissues derived from a normal mouse (non-transgenic mouse) as a control are shown on the left for comparison.
Fig. 9 is a set of graphs showing glomerular hypertrophy in megsin/RAGE/iNOS-Tg (triple-Tg) mice (16 weeks old). In the experimental animals of triple-Tg and comparative control, (a) size of glomerular tuft region, (b) ratio of mesangial matrix to glomerular tuft region, and (c) number of nuclei used to count glomerular cells in each experimental animal are shown.
Fig. 10 is a set of graphs showing glomerular hypertrophy in megsin/RAGE/iNOS-Tg (triple-Tg) mice (8 weeks old). In the experimental animals of triple-Tg and comparative control, (a) size of glomerular tuft region, (b) ratio of mesangial matrix to glomerular tuft region, and (c) number of nuclei used to count glomerular cells in each experimental animal are shown.
Fig. 11 is a set of immunohistological staining images showing glomerular and tubulointerstitial damages in a megsin/RAGE/iNOS-Tg (triple-Tg) mouse. (a) image of immunostaining using "α-SMA" (a marker of mesangial matrix activation and tubulointerstitial damage); (b) immunostaining image of macrophage infiltration using F4/80.
Fig. 12 is a graph showing the results obtained by measuring the increase of oxidative stress in megsin/RAGE/iNOS-Tg mice, using 8-OHdG (an oxidative stress marker).

### Best Mode for Carrying Out the Invention

The disease model animals of the present invention comprise nonhuman mammals expressing at least three genes: megsin gene, a gene encoding a receptor for advanced glycation end-products (RAGE), and an inducible nitric oxide synthase gene (iNOS). By expressing these three genes, marked symptoms of kidney disorders can be induced at an early stage as compared with conventional model animals. Such symptoms include marked renal hypertrophy with increased kidney-to-body weight ratio (1.5 to 2 times greater than that of megsin Tg mice or more and 1.5 times greater than that of normal mice or more), elevated urine albumin level (two times greater than that of RAGE/iNOS-Tg mice or more), and elevated blood triglyceride level (two times higher than that of megsin Tg mice or more). In addition, these three genes enable induction of tubulointerstitial disorders at an earlier stage than conventional megsin Tg mice and RAGE/iNOS-Tg mice, by altering the mesangial matrix, such as mesangial matrix expansion, increased accumulation of immunoglobulin and/or complement, and increases of collagen, laminin, and/or fibronectin, as well as tubulointerstitial fibrosis, infiltration of inflammatory cells, and such. Furthermore, the disease model animals of the present invention expressing megsin/RAGE/iNOS also exhibit low body weight (hypogenesis), hyperglycemia, and hypoinsulinemia when compared with normal animals.

The disease model animals of the present invention expressing megsin/RAGE/iNOS can be obtained, for example, by administering a substance that can act on the promoter of each endogenous gene (megsin, RAGE, or iNOS gene) of the animals. Preferably, the model animals can be obtained by creating triple transgenic animals introduced with the above-described three genes.

The megsin gene herein refers to a gene that is specifically expressed in mesangial cells as described above and, for example, can induce a pathological condition of kidney failure, such as mesangial proliferative glomerulonephritis, if introduced into mice alone. As an example of the megsin gene, human megsin cDNA is shown in SEQ ID NO: 1. The megsin gene of the present invention may be a megsin gene comprising a sequence similar to the above-mentioned human megsin gene shown in SEQ ID NO: 1, a nonhuman megsin gene, or such, as long as it can induce the types of nephritis as described above. For example, rat and mouse megsins are described in International Publication NO. WO 99/15652.

RAGE is a cell surface receptor that specifically recognizes irreversible advanced glycation end-products (AGE) formed by glycation in blood or tissues. A human RAGE cDNA encoding RAGE is shown in SEQ ID NO: 2. However, the RAGE gene of the present invention is not limited to the human RAGE gene shown in SEQ ID NO: 2, and may be a similar sequence that encodes a receptor protein capable of recognizing AGE, a human gene different from the sequence of SEQ ID NO: 2, a nonhuman RAGE gene, or the like.

The iNOS gene encodes an inducible nitric oxide synthase. The mouse cDNA has been deposited under GeneBank/EMBL Data Bank M84373, and herein the sequence is included as SEQ ID NO: 3. However, the sequence is not limited to the sequence of SEQ ID NO: 3 of the present invention, and it may be a similar sequence that differs from the sequence but encodes an inducible nitric oxide synthase, a functionally equivalent mouse gene, an iNOS gene derived from another species such as human or such.

The respective genes described above can be obtained based on the respective nucleotide sequence described above by conventional methods. For example, in the case of megsin gene, a cDNA encoding megsin gene can be isolated by screening the cDNA library of mesangial cells using a DNA comprising the nucleotide sequence shown in SEQ ID NO: 1 as a probe. Alternatively, a megsin cDNA can be obtained by PCR amplifying the megsin gene using the cDNA library as template, and primers designed based on the nucleotide sequence shown in SEQ ID NO: 1.

The RAGE gene is expressed in many cell types, such as vascular endothelial cell, smooth muscle cell, pericyte, and kidney mesangial cell. Therefore, a cDNA encoding a RAGE gene can be isolated by screening the cDNA library of any of these cells, using a DNA comprising the nucleotide sequence shown in SEQ ID NO: 2 as a probe. Alternatively, a RAGE cDNA can be obtained by PCR amplifying a RAGE gene using the cDNA library as template and primers designed based on the nucleotide sequence of SEQ ID NO: 2.

In the case of iNOS gene, a cDNA encoding an iNOS gene can be isolated by screening the cDNA library of, for example, IL-1β-stimulated pancreatic β cells or activated macrophages using a DNA comprising the sequence of SEQ ID NO: 3, or a portion thereof, as a probe. Alternatively, an iNOS cDNA can be obtained by PCR amplifying an iNOS gene using the cDNA library as template and primers designed based on the sequence of SEQ ID NO: 3.

A functionally equivalent megsin gene with a sequence different from the above sequence of SEQ ID NO: 1, a functionally equivalent RAGE gene with a sequence different from the sequence of SEQ ID NO: 2, and a functionally equivalent iNOS gene with a sequence different from the sequence of SEQ ID NO: 3 can be obtained by selecting sequences capable of hybridizing under stringent conditions to a corresponding sequence described above. For washing, conditions of stringency include standard conditions of "1x SSC, 0.1 % SDS, and 37°C"; more stringent conditions of "0.5x SSC, 0.1% SDS, and 42°C"; and even more stringent conditions of "0.1x SSC, 0.1% SDS, and 55°C".

In the creation of transgenic animals, it is beneficial to ligate each of the genes described above with a promoter that can express the gene in animal cells to be introduced with. The chicken β actin promoter which can express foreign genes in a broad range of vertebrates, including mice and rats, can be used. Alternatively, when a gene is to be expressed in a specific tissue, a tissue-specific promoter may be used. For example, the mouse flk-1 is a vascular endothelial cell-specific promoter. If required, an enhancer may be used in combination to enhance the expression of a foreign gene. A conventional vector (for example, pCAGGS or such) that comprises an enhancer and a promoter, and downstream thereof, a multi-cloning site for insertion of foreign genes may be used. In these vectors, a rabbit β globin terminator is placed downstream of the multi-cloning site, and this improves the expression efficiency of the inserted foreign genes.

Transgenic animals are created using each of the genes ligated with a promoter described above. The disease model animals of the present invention comprise a triple transgenic animal introduced with the three genes described above (megsin, RAGE, and iNOS genes). The triple transgenic animal may be created by introducing these three genes into one germinal cell and strongly expressing the three genes. Alternatively, each gene may be separately introduced into a different germinal cell and then the resulting respective transgenic animals are crossed sequentially to create a triple Tg animal having the three foreign genes. The method for creating the transgenic animal of the present invention is not limited to specific methods, and art-known methods for creating transgenic animals (see, for example, "Hassei Kohgaku Jikken Manyuaru (Manual for Experiments in Development Engineering)", ed. M. Katsuki (Japan, Kodansha), 1989; "Shin Seikagaku Jikken Kohza, Doubutsu Jikkenhou (New series, Lecture for Biochemical Experiments: Methods of Animal Experiments)" ed. The Japanese Biochemical Society, (Japan, Tokyo Kagakudojin) 1991) may be used. A general protocol for transgenic animal preparation is briefly described below.

Transgenic animals can be created by introducing genes into, for example, germinal cells comprising unfertilized eggs, fertilized eggs, sperms, and primordial cells thereof. As a cell to be introduced with the genes, a cell at the stage of embryogenesis, more specifically, a cell at the single cell stage or fertilized egg stage, and typically at the 8-cell stage or earlier in the development of a nonhuman mammal is employed. As a method for introducing the genes described above, calcium-phosphate method, electronic pulse method, lipofection method, agglutination method, microinjection method, particle gun method, DEAE-dextran method and such are known, and any of these methods may be used. When a transgenic animal is created for each gene, the genes are introduced independently into a different fertilized egg or such. Alternatively, when the three genes are co-introduced, a solution comprising the three genes is introduced into a fertilized egg by any one of the methods described above.

Here, the cells to be introduced with the genes may be cells derived from any nonhuman mammals that allow creation of transgenic animals. Specifically, it is possible to use cells from mice, rats, hamsters, guinea pigs, rabbits, goats, sheep, pigs, bovines, dogs, cats, or such. For example, in the case of rats, fertilized eggs into which genes can be introduced can be collected by crossing a normal male rat with a female rat administered with an ovulation inducing agent. In general, when a rat fertilized egg is used, the gene (or genes) can be introduced into the male pronucleus by microinjection. The cells into which the gene (or genes) has been introduced are cultured for about one night *in vitro.* Then, some cells into which the gene is expected to have been successfully introduced are transplanted into the oviduct of a surrogate mother, and then transgenic chimera animals are born. A pseudopregnant female prepared by mating with a male whose seminal duct has been incised is used as the surrogate mother.

The newborn transgenic chimera animals are tested to confirm whether the foreign gene (or foreign genes) has been integrated into the genome by analyzing genes in their somatic cells. The transgenic chimera animals are crossed with normal animals to give birth to F1 animals. When crossing, it is desirable to select individuals carrying a higher gene copy number. Typically, a foreign DNA to be introduced as a gene is integrated into the genome at the same site in multiple-copy tandem arrays. In general, as the copy number of an integrated gene becomes greater, the expression level of the gene is higher and a more distinct expression type can be expected. In addition, a relative comparison of copy number can be achieved by using dot blotting.

Among F1 animals that are born as a result of the crossing, animals carrying the foreign gene (or foreign genes) in their somatic cells are heterozygous transgenic animals and can pass the foreign gene (or foreign genes) onto germ cells. Thus, homozygous animals retaining the foreign gene (or foreign genes) homozygously can be obtained as F2 animals, by selecting animals carrying in their somatic cells the foreign gene (or the foreign genes) from the parent F animals, and allowing the selected animals to give birth to F2 animals.

When a transgenic animal is created for each gene by the method described above, first, two of these transgenic animals are crossed to create a double transgenic animal. A triple transgenic animal is created by crossing the resulting transgenic animal with an uncrossed transgenic animal. The disease model animals of the present invention may be transgenic animals of any generation as long as the animals express the three genes described above. The F1 generation that carries the three DNAs heterozygously and the F2 generation or a subsequent generation carrying the DNAs homozygously are also included in the present invention.

The triple Tg animals created as described above efficiently exhibit hyperglycemia and pathological conditions of kidney failure (in particular, hyperglycemic nephropathy) in early stages. For example, when a triple transgenic mouse of the present invention is compared with a conventional RAGE/iNOS transgenic mouse, the mouse of the present invention can develop diabetic nephropathy at about 16 weeks while it takes the conventional mouse about 40 weeks to develop it. As seen from the mouse example described above, the onset of the disease can be accelerated according to the present invention and thus the invention enables the disease model animals to be quickly provided. Thus, the triple transgenic mouse of the present invention can be used as a model for diabetic nephropathy when it is 14 weeks or older, preferably 16 weeks or older. In addition, in view of the fact that the conventional RAGE/iNOS transgenic mouse cannot develop diabetic nephropathy until about 40 weeks old, the triple transgenic mouse of the present invention is featured by its capability to serve as a diabetic nephropathy model at 14 to 40 weeks, more preferably at 16 to 40 weeks.

In addition, when compared with the conventional RAGE/iNOS-Tg and megsin Tg, the triple transgenic animals of the present invention exhibit homogeneous phenotypes, such as kidney function disorder, and thus model animals that are qualitatively more useful can be provided. Specifically, renal hypertrophy, increase in the urine albumin level, increase in the blood triglyceride level, and increase in the urine level of 8-OHdG, which is an oxidative stress marker, are detected in the triple transgenic animals of the present invention. Furthermore, in addition to proliferation of mesangial matrix and conditions of nodular glomerulosclerosis, type IV collagen, laminin, and fibronectin are increased, and the accumulation of immunoglobulins and complements is enhanced in the mesangial matrix. Tubulointerstitial fibrosis and infiltration of inflammatory cells are also observed. Such phenotypes make the model of diabetic nephropathy extremely useful.

The clinical and pathological features of kidney function disorder of the present invention are described below. The disease model animals of the present invention expressing megsin/RAGE/iNOS exhibit renal hypertrophy. The renal hypertrophy is assessed based on the increase of kidney-to-body weight ratio (KWBW). Weights of the body and kidney are measured, and KWBW is calculated based on these values. When the KWBW value is greater than that of a normal animal, the kidney is judged to be hypertrophic.

Proteinuria is also a characteristic feature of the disease model animals of the present invention expressing megsin/RAGE/iNOS. In diabetic nephropathy, a trace amount of albuminuria is detected at early stages of nephropathy and then persistent proteinuria is detected as the disease advances. The animals of the present invention also exhibit such a characteristic feature of proteinuria. Proteinuria refers to a symptom in which the level of protein excreted into urine is higher than the animal's normal level. Methods for assaying urine protein are known in the art. For example, biochemical procedures, such as protein-error method and dye-binding method, are widely used as semi-quantitative methods for assaying urine protein. Urinary test papers based on these assay principles are commercially available. An immunological principle-based assay has been used in practice as a more sensitive and specific method for assaying urine protein. In an immunological assay, for example, albumin excreted into urine is detected using an antibody against serum albumin. Normally, only a trace amount of protein is excreted into urine. For example, in rats, the normal level of protein in urine is considered to be 0-40 mg/dl. Thus, a rat is assessed to be exhibiting proteinuria when protein is persistently excreted into urine at levels higher than the above value. In the present invention, there is no limitation on the type of proteins excreted into urine. In general, urine protein mainly comprises major blood proteins: albumin and globulin.

The blood triglyceride level is elevated in the megsin/RAGE/iNOS-expressing animals of the present invention. Blood triglyceride levels are known to be elevated secondarily in patients with diabetes mellitus or kidney disorder. Such condition is also observed in the animals of the present invention. Increase in the blood triglyceride level refers to a level that exceeds the animal's normal range. Methods for assaying blood triglyceride are known in the art. For example, it is possible to use the LPL/GK/GPDH-diaphorase/formazan dye method.

Expansion of mesangial matrix and nodular glomerulosclerosis occur in the megsin/RAGE/iNOS-expressing animals of the present invention. Thickening of glomerular basement membrane, expansion of mesangial matrix, and nodular sclerotic lesion in glomerulus are characteristic of diabetic nephropathy. These characteristic features are also exhibited in the model animals of the present invention. Furthermore, the expansion of mesangial matrix can be observed based on the increase of collagen, laminin, fibronectin or such generated during the process of expansion. Pathological tissues can be immunohistologically analyzed using the respective antibodies. If the immunohistological images of the animal have a wider area and/or stronger intensity of staining as compared with its normal immunohistological images, it is determined that collagen, laminin, fibronectin or such has been increased. Meanwhile, thickening of glomerular basement membrane can be detected through pathological analysis using an electron microscope. A major cause of basement membrane thickening is the hyperglycemia-dependent abnormality of matrix metabolism (enhanced production and depressed metabolism), and it has been understood that the underlying mechanism is the same as that for the proliferation of mesangial matrix.

The model animals of the present invention expressing megsin/RAGE/iNOS are also characterized by enhanced accumulation of immunoglobulins and complements in the mesangial matrix. In human diabetic nephropathy, the deposition of immunoglobulins and complements is detected in the glomerular capillary walls and mesangial region where mesangial matrix exists. Such pathological changes also appear in the model animals of the present invention. Immunoglobulins and complements can be detected by immunohistological staining using antibodies that are specific to them. If the area or intensity of staining is wider or stronger than those in the immunohistological staining images of the animal's normal tissues, the accumulation of immunoglobulins and complements is judged to be enhanced.

The megsin/RAGE/iNOS-expressing model animals of the present invention can also be characterized by tubulointerstitial fibrosis and infiltration of inflammatory cells. Such findings of the renal tubule and interstitium are also observed in the late-stage pathological conditions of advanced human diabetic nephropathy. Such pathologies also appeared in the model animals of the present invention. Tubulointerstitial fibrosis can be detected by immunohistological staining using a specific antibody against α-SMA. Fibrotic cells can be detected by using a specific antibody against α-SMA to visualize cells that exhibit positive reaction in the renal tubule or interstitium. Alternatively, fibrosis can be semi-quantified by directly scoring the degree of damage in the renal tubule or interstitium under a microscope. Meanwhile, infiltration of inflammatory cells such as macrophages in the tubular interstitium can be detected by immunohistological staining using antibodies or such that are specific to the inflammatory cells. For example, F4/80 described in the Examples can be used as an antibody to detect macrophages.

The megsin/RAGE/iNOS-expressing model animals of the present invention can also be characterized by an increase in the oxidative stress. Recent analyses of the pathological state of human diabetic nephropathy have suggested that the onset of diabetic nephropathy is caused by oxidative stress. The increase of oxidative stress as observed in such pathological state of human diabetic nephropathy is also observed in the model animals. The oxidative stress can be measured, for example, using animal urine or blood. For example, 8-OHdG, an oxidative stress marker, can be easily measured using animal urine as a sample and the commercially available ELISA kit or such.

As described above, the model animals of the present invention have characteristic features of kidney function disorders such as diabetic nephropathy, glomerular disorder (nodular sclerotic lesion), and tubulointerstitial disorder, and thus can serve as useful model animals for these diseases. In addition, the model animals of the present invention exhibit hyperglycemia and hypoinsulinemia at early stages, and thus are useful as model animals for diabetes mellitus. Thus, the model animals of the present invention can be used in studies to elucidate the onset mechanisms of diabetes mellitus and complications thereof including diabetic nephropathy and nodular glomerulosclerosis, and for other purposes. Also, they are useful as an evaluation system for developing therapeutic methods, agents, and such, for hyperglycemia and kidney function disorders.

The present invention also provides methods for evaluating therapeutic agents against hyperglycemia or kidney function disorders, which employ the disease model animals described above. The first evaluation method comprises the following steps of:
(1) administering a test compound to an above-described megsin/RAGE/iNOS-expressing disease model animal which comprises a nonhuman mammal; and
(2) testing the kidney function in the above-described disease model animal to which the test compound has been administered.

In the above evaluation method, first, a test compound is administered to a megsin/RAGE/iNOS-expressing model animal of the present invention. There is no limitation on the administration route. It is possible to select oral administration, intravenous injection, intraperitoneal administration, or such, or any alternative method that is adequate for the test compound. The disease model animal treated by the administration, and a control animal, which is an identical disease model animal that has not been administered with the test compound, are tested for their kidney functions using kidney function markers. For example, the following kidney function markers are known and can be used in the evaluation method. Kidney function markers: blood creatinine and urea nitrogen, urine hemoglobin, albumin, β2-microglobulin, and α1-acid glycoprotein. Methods for measuring the kidney function markers are also known in the art.

Thus, efficacy of the test compound as a therapeutic agent can be evaluated by observing these indices before and after administration of the test compound and comparing the results obtained. Alternatively, when transgenic animals derived from the same line are used, the efficacy can also be compared between test compounds by observing these indices in the animals and comparing the results obtained.

The second evaluation method uses as an indicator a clinical marker associated with glomerular disorders, such as diabetic nephropathy, comprising the steps of: administering a test compound to the above-described megsin/RAGE/iNOS-expressing disease model animal and measuring at least any one of the kidney-to-body weight ratio, proteinuria level, and blood triglyceride in the disease model animal after administration of the test compound. Specifically, in this method, the therapeutic effect on kidney function disorders is evaluated based on one or more indicators of increased kidney-to-body weight ratio, proteinuria, and blood triglyceride.

In the second evaluation method described above, first, a test compound is administered to a megsin/RAGE/iNOS-expressing model animal of the present invention. As described above, there is no particular limitation to this route. The kidney functions of the disease model animal treated by the administration and a control model animal that has not been administered with the test compound are compared based on the indices described above. Furthermore, the model animal may be compared with healthy animal subjects. The method for measuring the increase in kidney-to-body weight ratio, proteinuria, and blood triglyceride is the same as described above. In comparison with an untreated group, a test compound which can relieve kidney disorder or restore kidney function to the level of a normal animal when administered can be a therapeutic agent for accompanying kidney function disorders of glomerular failure, such as diabetic nephropathy.

The third evaluation method comprises the steps of: administering a test compound to a megsin/RAGE/iNOS-expressing disease model animal of the present invention, and evaluating whether the mesangial matrix has been altered, or whether the degree of alteration has been reduced by administration of the test compound. In this evaluation method, the relieving effect on kidney function disorder is evaluated by histological examination of the mesangial matrix. In accompanying kidney function disorders of glomerular failure, such as diabetic nephropathy, in particular, expansion of mesangial matrix, enhanced accumulation of immunoglobulins and/or complements, increases of collagen, laminin, and/or fibronectin are seen. Thus, evaluation is achieved based on the relief or recovery from any one of these.

More specifically, a test compound is administered to a megsin/RAGE/iNOS-expressing model animal of the present invention. There is no particular limitation to the administration method. The method can be appropriately selected for the test compound. The mesangial matrices of the disease model animal treated by the administration and a control model animal that has not been administered with the test compound are compared histologically. For example, whether the mesangial matrix expansion is relieved is evaluated by measuring the area of mesangial matrix stained by PAS. In comparison with an untreated group, if the area of mesangial matrix in a group administered with the test compound is reduced or restored to the level of a normal animal, the test compound is evaluated to have therapeutic effects on accompanying kidney function disorders of glomerular failure, such as diabetic nephropathy. Likewise, the mesangial matrix is immunostained using a specific antibody against immunoglobulin or complement to determine the quantity of accumulated immunoglobulin or complement. When the value of a group administered with the test compound is smaller than that of an untreated group, the test compound is evaluated to have therapeutic effects on accompanying kidney function disorders of glomerular failure. Alternatively, mesangial matrix is immunostained using a specific antibodies against collagen, laminin, and/or fibronectin to determine their contents. If the value of a group administered with the test compound is smaller than that of an untreated group, the test compound is evaluated to have therapeutic effects on accompanying kidney function disorders of glomerular failure.

The fourth evaluation method comprises the steps of: administering a test compound to a megsin/RAGE/iNOS-expressing disease model animal of the present invention, and evaluating whether the tubular interstitium of the disease model animal has been altered or whether the degree of alteration has been reduced after administration of the test compound. In this evaluation method, the relieving effect on kidney function disorder is evaluated through histological examination of the tubular interstitium. This evaluation method examines whether the pathological state has been relieved or recovered, based on the pathological state of tubular interstitium observed in the late-stage pathological conditions of advanced human diabetic nephropathy.

Specifically, a test compound is administered to a megsin/RAGE/iNOS-expressing model animal of the present invention. The tubular interstitium of the disease model animal treated by the administration and a control model animal that has not been administered with the test compound are compared histologically. For example, tubulointerstitial fibrosis can be quantified by histological staining using the above-described antibody against α-SMA or by direct microscopic examination. Then, if the degree of tubulointerstitial fibrosis in a group administered with the test compound is reduced in comparison with an untreated group, or if the fibrosis becomes undetectable as in the normal animal, the test compound is evaluated to have therapeutic effects on accompanying kidney function disorders of glomerular failure, such as diabetic nephropathy. Likewise, tubular interstitium is immunostained using a specific antibody against inflammatory cells, such as macrophage, to determine the proportion of inflammatory cells present. If the measured value of a group administered with the test compound is smaller than that of an untreated group, the test compound is evaluated to have therapeutic effects on accompanying kidney function disorders of glomerular failure.

The present invention also provides methods for evaluating the therapeutic effect on diabetes mellitus. This method comprises the steps of: (1) administering a test compound to the above-described megsin/RAGE/iNOS-expressing disease model animal; and (2) determining the glucose level and/or insulin level of the disease model animal after administration of the test compound.

In the evaluation method of the present invention, the degree of therapeutic effect on diabetes mellitus can be evaluated using a marker of diabetes mellitus, for example, glucose or insulin level, as an indicator. The subject of glucose level determination may be any type of body fluid, including blood, urine, and sweat. When insulin level is to be determined, the preferred sample is blood. Alternatively, hemoglobin Hcl, glycated albumin, fructosamine, urine ketone body, or the like can be used as an index to evaluate the therapeutic effect on diabetes mellitus symptoms. The therapeutic effect on diabetes mellitus can be evaluated by monitoring changes of these diagnostic indices over time. Thus, efficacy of a test compound as a therapeutic agent can be evaluated by observing these indices before and after administration of the test compound and comparing the results obtained. Alternatively, when transgenic animals derived from the same line are used, the efficacy can also be compared between test compounds by observing the indices between the animals and comparing the results obtained.

All prior art documents cited herein are incorporated herein by reference.

Herein below, the present invention will be specifically described with reference to Examples, but it is not to be construed as being limited thereto.

### [Example 1] Preparation of megsin/RAGE/iNOS-Tg

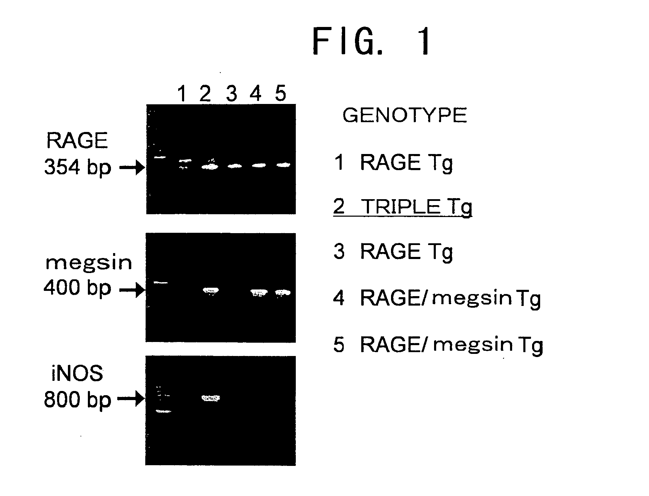

RAGE/iNOS-Tg mice (Yamamoto, Y, et al., J. Clin. Invest., 108:261-268 (2001)) were crossed with megsin-Tg mice (Miyata, T., et al., J. Clin. Invest., 109: 585-593 (2002)) to obtain megsin/RAGE/iNOS-Tg mice. Genomic DNA was extracted from a tail tissue, and the expressions of RAGE, iNOS, and megsin were confirmed by PCR according to the method described in the documents (Yamamoto, Y., et al., J. Clin. Invest., 108:261-268 (2001); Miyata, T., et al., J. Clin. Invest., 109: 585-593 (2002)). Specifically, a part of the tail of a Tg mouse was excised 4 weeks after birth or later, and the genomic DNA was extracted using a DNA extraction kit (Qiagen tissue kit; Qiagen). Using the genomic DNA as template, fragments of the introduced genes were amplified by PCR to confirm the presence of each introduced gene. For the PCR detection of megsin, β-gl-3 primer (5'-CTT CTG GCG TGT GAC CGG CG-3'/SEQ ID NO: 4) and hM2-2 primer (5'-ATC GAA TTC TGA GAT CAT AAT CCC TGT GGG ATG C-3', SEQ ID NO: 5) were used, and individuals in which PCR amplification products (400 bp) were obtained by using these primers (Fig. 1) were selected. For the detection of RAGE gene, flk-1 promotor primer (5'-AGG GAC GGA GAA GGA GT-3'/SEQ ID NO: 6; Ronicke, V. et al., Circ. Res., 79:277-285 (1996)) and human RAGE gene primer (5'-TCACCCCACAGACTGAG-3'/SEQ ID NO: 7; Sugaya, K. et al., Genomics, 23:408-419 (1994)) were used and an amplification product of 3 54 bp was detected (Fig. 1). For the detection of iNOS gene, F-primer (5'-GTGGGCTATGGGTTTGTGGAAGGAGA-3', SEQ ID NO: 8) and R-primer (5'-CGATGTCACATGCAGCTTGT-3'/SEQ ID NO: 9) were used and an amplification product of 800 bp was detected (Fig. 1). The resulting megsin/RAGE/iNOS-Tg mice were backcrossed with non-transgenic CD1 mice (Charles River Japan, Inc.) to have a CD-1 genetic background.

### [Example 2] Pathological findings in megsin/RAGE/iNOS-Tg

Kidneys were excised from 16-week-old megsin /RAGE/iNOS-Tg mice, and PAS staining and PAM staining of the glomeruli were carried out according to a conventional method and the method described in WO 01/24628. Tissues that were fixed with Carnoy's fixative, embedded in paraffin, and sliced into 4-micron sections were used in the PAM staining. PAS staining (Periodic Acid Schiff reaction) generally stains polysaccharides red. In kidney pathology, proliferation of mesangial matrix and thickening of basement membrane can be observed by staining glycoproteins in red, which are components of the mesangial matrix and basement membrane. Meanwhile, in PAM staining (Periodic Acid-Methenamine Silver stain), components of the basement membrane and connective tissues are generally stained in black (silver impregnation). In kidney pathology, sclerosis (fibrosis) of mesangial matrix and thickening of basement membrane are stained in black.

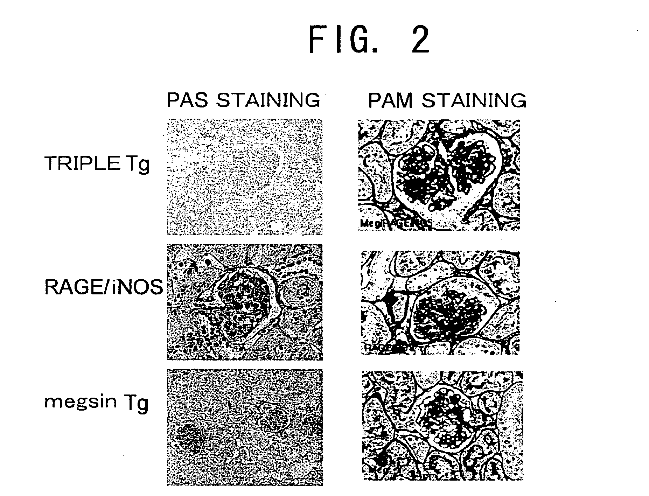

The results of PAS staining and PAM staining are shown in Fig. 2. According to the results of PAS staining, severe glomerular disorder (nodular sclerotic lesion) was found in all cases of the hyperglycemia group (6 mice; 500 mg/dl or higher blood glucose level) among 9 megsin/RAGE/iNOS-Tg mice. No abnormality was found in the wild-type mice. Specifically, a marked expansion of the mesangial matrix was found dispersedly throughout the region, and glomerular hypertrophy was also detected. Meanwhile, an image of rather reduced glomerular cell count and glomerular sclerosis accompanied by glomerular cell death was shown. In addition, sclerosis of the expanded mesangial matrix was also confirmed by PAM staining.

Glomeruli exhibiting global or segmental expansion of mesangial matrix were found locally in the control RAGE/iNOS-Tg mice (11 mice; 10 of them are from the hyperglycemia group (400 mg/dl or higher)). The glomerular cell count remained unchanged. Tubulointerstitial alterations were rarely found. The sclerosis detected by PAM staining was milder than that in the RAGE/iNOS/megsin-Tg mice. Furthermore, no particular pathological finding was detected in the megsin-Tg mice at 16 weeks (Fig. 2).

### [Example 3] Kidney weight and biochemical tests of urine and blood in megsin/RAGE/iNOS-Tg

Kidney weight and biochemical parameters of urine and blood in 16-week-old megsin/RAGE/iNOS-Tg mice were measured (Table 1). Other Tg mice (iNOS-Tg, RAGE/iNOS-Tg, megsin/iNOS-Tg, megsin/RAGE-Tg, megsin-Tg, and RAGE-Tg) and wild-type mice (CD-1) were used as a control for comparison. The items to be measured are as follows: body weight (B W); kidney weight (KW); serum: total protein (TP: Biuret method); triglyceride (TG: LPL/GK/GPDH-diaphorase/formazan dye method); total cholesterol (Tcho: enzyme method); urea nitrogen (BUN: urease-ultraviolet method); creatinine (Cr: alkaline picrate method); insulin (insulin: ELISA); blood glucose level (GLU: GOD/POD method); GOT; and GPT; urine: albumin (HbA1c: latex agglutination inhibition method); total protein (TP: pyrogallol red method); urea nitrogen (BUN: urease-ultraviolet method); and creatinine (Cr: alkaline picrate assay). The results of TP, CRE, and GLU as shown were measured using urine (one-time collection: spot urine) as a sample.

As shown in Table 1, the KW/BW of megsin/RAGE/iNOS-Tg mice was higher than that of the RAGE/iNOS-Tg mice and megsin-Tg mice, and renal hypertrophy was found in the megsin/RAGE/iNOS-Tg mice. In addition, albuminuria and increase in the blood triglyceride level were also found in the megsin/RAGE/iNOS-Tg mice. Furthermore, the megsin/RAGE/iNOS-Tg mice also exhibited underweight, hyperglycemia, and hypoinsulinism compared with non-transgenic mice.

**Table 1**

| Triple Tg Biochemical Data information: 16 - week -old , CD-1 (F4) | | | | | | | | |
|---|---|---|---|---|---|---|---|---|
| Mouse Line | HbA1c (%) | BW (g) | KW (g) | KW/BW | GLU (mg/dl) | Insul in (ng/ml) | BUN (mg/dl) | CRE (mg/dl) |
| NOS (n=5) | 7.2±1.3 | 43.5±3.3 | 1.08±0.15 *3 | 0.025±0.003 *1 | 858.0±200.8 *1 | 0.34±0.16 *2 | 41.8±12.7 | 0.78±0.11 |
| CD-1 (n=6) | | 43.1±8.8 | 0.72±0.14 | 0.017±0.003 | 194.7±56.8 | 4.40±3.80 | 30.7±5.0 | 0.62±0.13 |
| meg/NOS/RAGE (n=8) | 6.5±1.6 | 33.1±6.5 *1 | 0.98±0.32 | 0.030±0.009 *1 | 682.6±373.8 *1 | 0.43±0.37 *2 | 40.6±16.6 | 0.76±0.21 |
| RAGE/NOS (n=10) | 7.6±1.3 | 45.3±3.4 | 1.11±0.19 *3 | 0.024±0.004 *1 | 766.4±167.8 *1 | 0.27±0.20 *2 | 38.6±7.3 *2 | 0.75±0.15 |
| meg/NOS (n=5) | 5.9±2.7 | 35.6±5.7 *3 | 0.86±0.31 | 0.022±0.006 | 699.6±390.9 *1 | 0.52±0.52 | 41.2±19.3 | 0.68±0.22 |
| meg/RAGE (n=10) | | 40.2±10.2 | 0.65±0.14 | 0.016±0.004 | 191.6±87.6 | | 35.7±8.0 | 0.62±0.11 |
| meg (n=11) | | 44.2±11.4 | 0.69±0.17 | 0.015±0.002 | 225.9±64.8 | 2.64±2.32 | 34.3±5.5 | 0.60±0.07 |
| RAGE (n=11) | | 48.5±7.3 | 0.71±0.17 | 0.014±0.004 | 180.8±35.1 | 3. 99±3. 82 | 33.4±6.6 | 0.56±0.11 |
| | | | | | | | | |

| Mouse Line | TP (g/dl) | TCHO (mg/dl) | TG (mg/dl) | GOT (U/l) | GPT (U/l) | ALB/CRE Ratio | | |
|---|---|---|---|---|---|---|---|---|
| NOS (n=5) | 3.8±0.3 | 146.2±17.2 | 318.6±303.5 | 90.2±37.2 | 40.2±11.1 | | | |
| CD-1 (n=6) | 4.4±0.3 | 148.8±54.0 | 169.0±88.2 | 137.0±33.7 | 49.7±25.5 | 0.02±0.01 | | |
| meg/N0S/RAGE (n=8) | 4.2±1.6 | 145.5±29.3 | 436.3±296.1 | 149.3±34.0 | 41.1±11.6 | 0.14±0.07 | | |
| RAGE/NOS (n=10) | 4.7±2.2 | 148.2±18.1 | 388.3±258.3 | 109.3±47.9*2 | 48.7±22.9 | 0.06±0.02 | | |
| meg/NOS (n=5) | 4.0±0.5 | 151.6±31.8 | 500.6±402.5 | 105.4±32.5 | 38.8±15.2 | 0.15±0.06 | | |
| meg/RAGE (n=10) | 4.7±0.9 | 132.0±39.7 | 224.5±122.6 | 145.0±42.3 | 53.1±30.7 | 0.09±0.04 | | |
| meg (n=11) | 4.9±1.7 | 161.4±35.6 | 221.1±72.5 | 140.0±79.0 | 48.2±18.5 | - | | |
| RAGE (n=11) | 4.3±0.3 | 137.5±31.7 | 190.5±105.2 | 160.9±100.4 | 50.2±25.2 | 0.11±0.02 | | |

| | | | | | | | | |
|---|---|---|---|---|---|---|---|---|
| *1 : *P* < 0.001, vs GD-1 Note 1: Measured by DR I CEHM 3500V (FUJIFILM MEDICAL CO., LTD.). *2 : *P* < 0.05, VS CD-1 Note 2: Data of insul in is value for reference because of a large number of hemolytic specimens. *3 : *P* < 0.01, vs CD-1 Note 3: "ALB/CRE ratio"of meg group can not be measured because of a shortage of specimens. non : NS | | | | | | | | |

### [Example 4] Deposition of immune complexes by immunofluorescence microscopy

The deposition of immune complexes was examined using immunofluorescence microscopy. Kidney tissues were collected from the megsin/RAGE/iNOS-Tg mice, embedded in a frozen-tissue embedding agent (OCT compound; Tissue Tek Miles, Elkhart, IN), and were rapidly frozen in dry ice/acetone. 4-µm frozen sections were prepared from the frozen embedded tissues. These frozen sections were treated with blocking in 4% skimmed milk at room temperature for 60 minutes, and then incubated at 4°C overnight with any one of 1:200 diluted fluorescein isothiocyanate (FITC)-labeled goat anti-mouse IgG, IgA, IgM, and C3 antibodies (Cappel Research Products). After the reaction, the sections were washed with PBS and mounted with glycerin-PBS, and then examined under a fluorescence microscope.

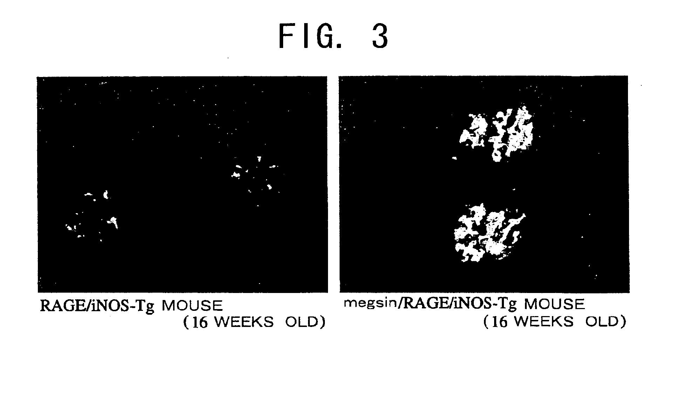

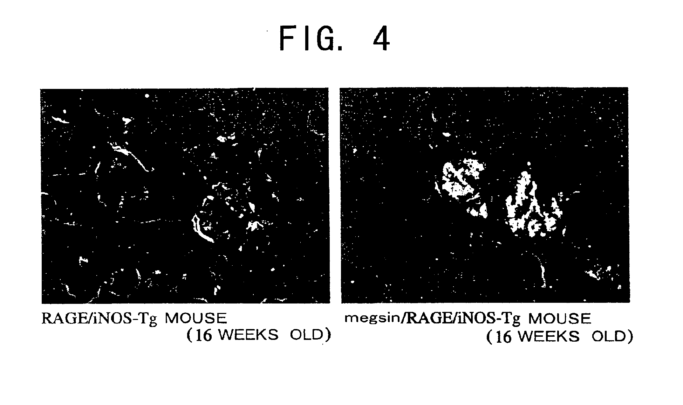

The results showed that the accumulation of immunoglobulin and complement in mesangial matrix had increased in the megsin/RAGE/iNOS-Tg mice as compared with RAGE/iNOS-Tg mice (Figs. 3 and 4).

### [Example 5] Changes in the mesangial matrix

The Tg mice were examined for changes in their mesangial matrices at 16 weeks by immunohistochemical staining. To identify the components of mesangial matrix, frozen sections were treated by an immunohistological staining method (Nangaku, M. et.al., J. Am. Soc. Nephrol. 10, 2323-2331, 1999) using the antibodies indicated below. Collagen type IV was identified using a goat anti-collagen type IV polyclonal antibody (Southern Biotechnology); fibronectin was identified using a rabbit anti-fibronectin antibody (Chemicon); and laminin was identified using a rabbit anti-laminin antibody (Chemicon).

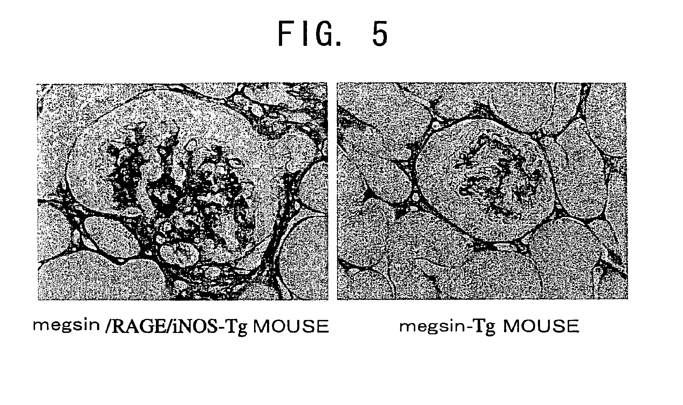

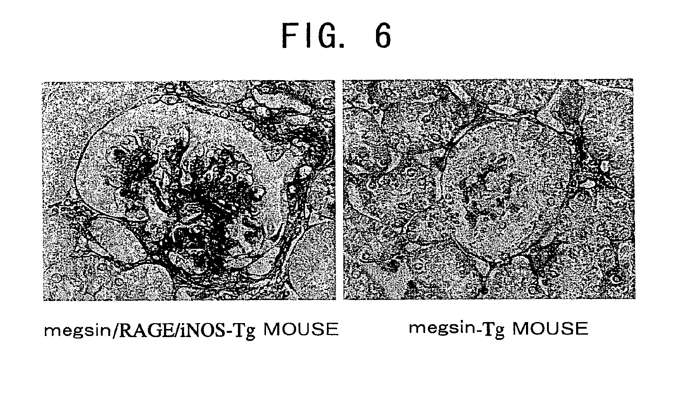

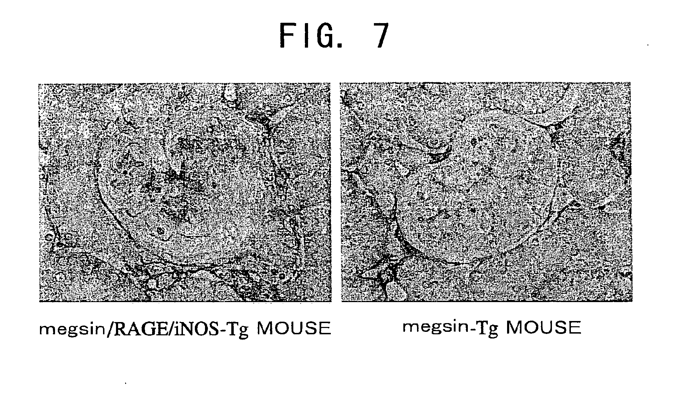

As compared with the RAGE/iNOS-Tg and megsin-Tg control mice, type IV collagen, laminin, and fibronectin were found to be increased in the expanded mesangial matrices of the megsin/RAGE/iNOS-Tg mice (Figs. 5, 6, and 7). Such a phenomenon is also known to be detected in human diabetic nephropathy.

In the transgenic animals of the present invention, the remodeling of a matrix that has expanded due to megsin overexpression was impaired rapidly, and this aggravated the glomerular disorder and shortened the time until the onset of sclerosis. It is also presumed that the glomeruli of the megsin/RAGE/iNOS-Tg animals are composed of mesangial cells exposed to burdens such as high megsin expression and hyperglycemia, and endothelial cells under oxidative stress. When only the mesangial cells were exposed to the burdens of megsin overexpression and hyperglycemia, the glomerular disorder is very mild (megsin/iNOS-Tg). However, if the endothelial cells are also exposed to burdens such as activation of the AGE-RAGE pathway, specifically, strong oxidative stress and various cytokines produced by endothelial cells, the clinical condition becomes more serious. This is supported by the fact that the clinical condition is very mild in the megsin/RAGE/iNOS-Tg mice (Tg mice in which endothelial cells are not under the burden of AGE-RAGE pathway activation) whose glucose level is normal. The megsin/RAGE/iNOS-Tg mouse model suggest that cross talk between the mesangial cells and endothelial cells influences the pathological development.

### [Example 6] Pathological analysis of the megsin/RAGE/iNOS-Tg mice

Pathological analysis of the kidney tissues of the megsin/RAGE/iNOS-Tg mice was carried out using an electron microscope. Kidney tissues from 16-week-old megsin/RAGE/iNOS-Tg mice were fixed by two hours of immersion in 0.1 M sodium phosphate buffer containing 2% glutaraldehyde, and then in 2% osmium tetroxide. The fixed kidney tissues were dehydrated with ethanol and at the end embedded in Epon 812 (TAAB; England). Ultra-thin sections excised from the samples were stained with uranium acetate and treated with acetone, and then analyzed using a transmission electron microscope (at 20,000 fold magnification; JEM-1200EX, JEOL LTD., Japan).

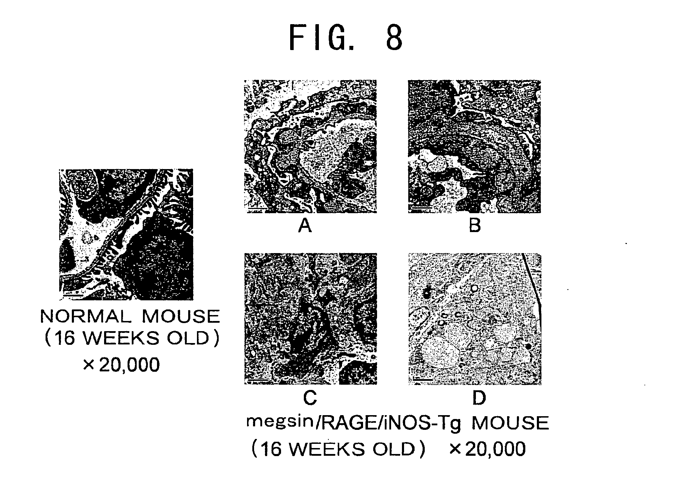

The analysis result is shown in Fig. 8. Loss of foot processes and high-density deposits in glomerular epithelial cells, and loss of fenestrae in glomerular endothelial cells was found in the megsin/RAGE/iNOS-Tg mice (Fig. 8A). In addition, irregular stratified glomerular basement membrane (Fig. 8B), proliferation of the mesangial matrix, and nodules (Fig. 8C), and also vacuole formation in the glomerular epithelial cells (Fig. 8D) were found in the megsin/RAGE/iNOS-Tg mice. These findings suggest that morphological abnormalities occurred in the cells that constitute the glomerulus (epithelium, endothelium, and mesangial cell) and basement membrane of the megsin/RAGE/iNOS-Tg mice.

### [Example 7] Acceleration of mesangial matrix expansion as a result of high megsin expression

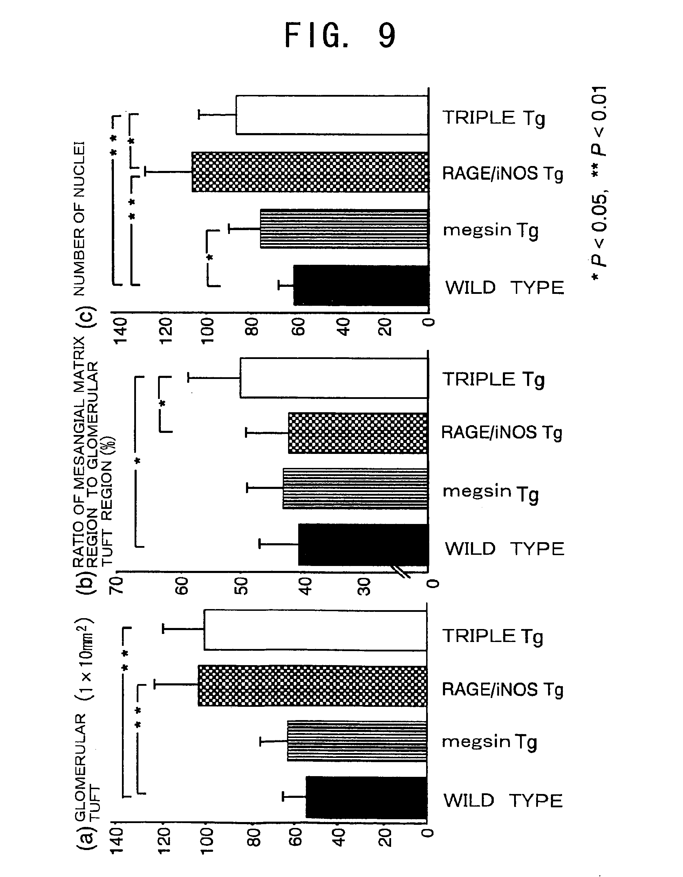

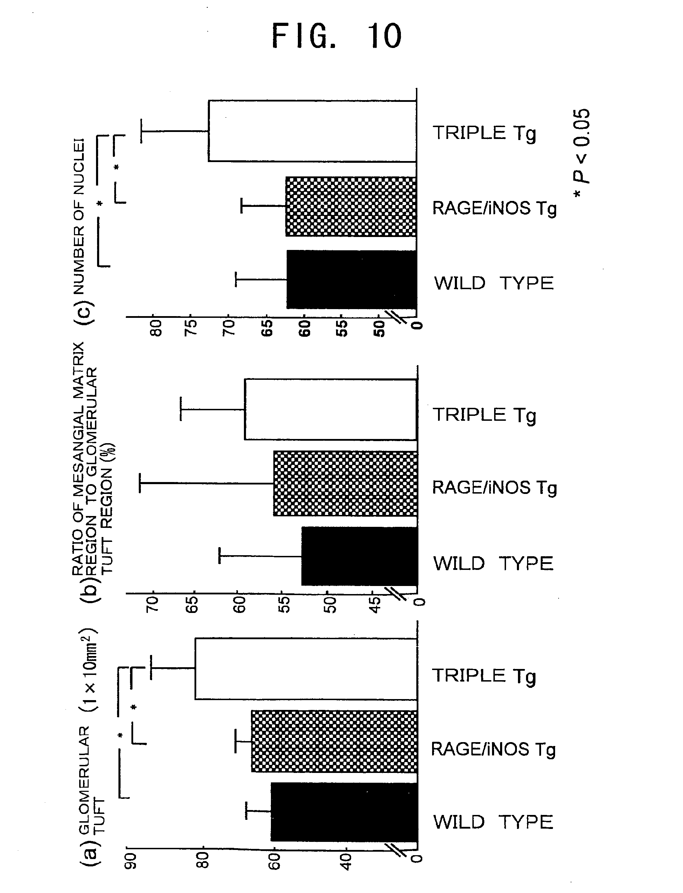

Glomerular lesions in 8- and 16-week-old megsin/RAGE/iNOS-Tg mice were examined by computer graphic analysis. Wild-type mice, megsin-Tg mice, and RAGE/iNOS-Tg mice of the same age were also analyzed by the same procedure. Specifically, PAS-stained kidney sections were prepared from each test mouse. Each staining image was scanned using a 3CCD camera (Olympus Optical Co., Tokyo, Japan), and the glomerular tuft region and mesangial region (a portion of glomerular tuft region) were examined and the glomerular cell count was determined by blind analysis using the Image Grabber PCI software (Fuji Photo Film Co., Tokyo Japan and Mac Aspect (Mitani Co., Tokyo, Japan). Twenty consecutive units of glomeruli in the midcortex were examined. Glomerular cross-sections containing only a small portion of the glomerular tuft (20 or less independent tubular segments contained in one cross section) were excluded from the subjects of the experiment. Furthermore, to avoid experimenter bias, both the greatest glomerulus and the smallest glomerulus were excluded from the 20 glomerulus test subjects. The remaining 18 glomeruli in each section were tested and the average was presented as "mean value +/- standard deviation". The analysis results obtained using the 16-week-old mice are shown in Fig. 9, and the results using the 8-week-old mice are shown in Fig. 10.

In comparison with the wild type and megsin-Tg mice, glomerular hypertrophy was found in both the RAGE/iNOS-Tg mice and the megsin/RAGE/iNOS-Tg mice at 16 weeks old (Fig. 9 (a)). High megsin expression did not accelerate glomerular hypertrophy in the RAGE/iNOS-Tg mice. At 16 weeks old, the wild-type mice, megsin-Tg mice, and RAGE/iNOS-Tg mice were revealed to be indifferent in the size of their mesangial matrices. However, expansion of the mesangial matrix was notably accelerated by high megsin expression in the megsin/RAGE/iNOS-Tg mice (Fig. 9(b)). These fmdings correlate with the development of severe mesangial matrix expansion and nodular lesion in the megsin/RAGE/iNOS-Tg mice in comparison with the RAGE/iNOS-Tg mice.

Meanwhile, hypertrophy of the glomerular tuft region in the Tg mice were detected at an early stage of 8 weeks, and the degree of hypertrophy was more serious in the megsin/RAGE/iNOS-Tg mice (Fig. 10(a)). There was no statistically significant difference in the ratio of mesangial matrix to glomerular tuft region among the wild type, RAGE/iNOS-Tg, and megsin/RAGE/iNOS-Tg mice of the same age, but there is a tendency of increasing ratio across the transgenic mice (Fig. 10(b)).

### [Example 8] Early-stage proliferation of glomerular cells by high megsin expression

The results of image analysis for the glomerular cell count described in Example 7 shows that the cell count increased in the megsin/RAGE/iNOS-Tg mice at an early stage of 8 weeks (Fig. 10(c)). Meanwhile, the cell count in the megsin/RAGE/iNOS-Tg mice was statistically significantly decreased as compared with the cell count in the 16-week old RAGE/iNOS-Tg mice (Fig. 9(c)). In addition, the glomerular cell count in any of the transgenic mice at 16 weeks old was greater than the glomerular cell count in the wild type mice of the same age.

### [Example 9] Glomerular or tubulointerstitial disorder in the megsin/RAGE/iNOS-Tg mice

Immunohistological staining was carried out for the detection of glomerular disorder and macrophage infiltration. 4-µm sections fixed with methyl Carnoy were stained with a mouse monoclonal antibody (400-times dilution; Boehringer Mannheim; Mannheim, Germany) against α-smooth muscle actin (α-SMA), which is a marker of mesangial cell activation and tubulointerstitial fibrosis, and a rat monoclonal antibody (400-times dilution; Caltag laboratories; Burlingame, CA, USA) against F4/80, which is a cell surface marker of mouse macrophage. Localization of the primary antibodies was visualized by an indirect immunoperoxidase method (Miyata T, Inagi R, et al., Overexpression of the serpin megsin induces progressive mesangial cell proliferation and expansion, JCI 109, 2002).

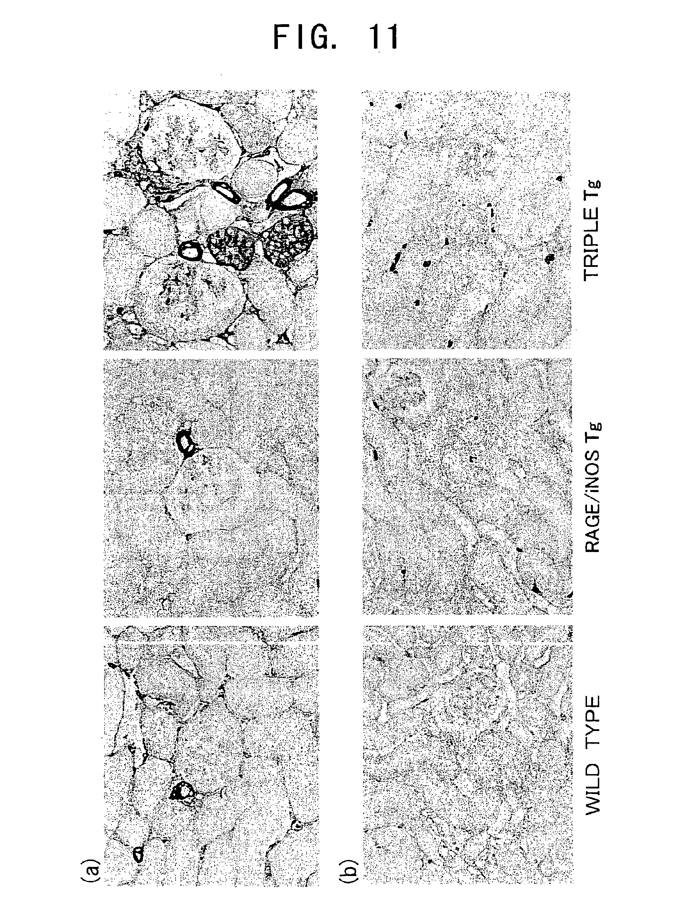

The glomerular disorder of the megsin/RAGE/iNOS-Tg mice was associated with mesangial cell activation, as shown by the immunostaining of α-SMA (Fig. 11 (a)). The de *novo* expression of α-SMA was detected in some fractions of the renal tubules and interstitial cells of the megsin/RAGE/iNOS-Tg mice, and this suggests tubulointerstitial fibrosis has occurred (Fig. 11 (a)). Infiltration of inflammatory cells into the tubular interstitium was detected in the megsin /RAGE/iNOS-Tg mice by F4/80 staining (Fig. 11(b)).

### [Example 10] Increase of oxidative stress in the megsin/RAGE/iNOS-Tg mouse

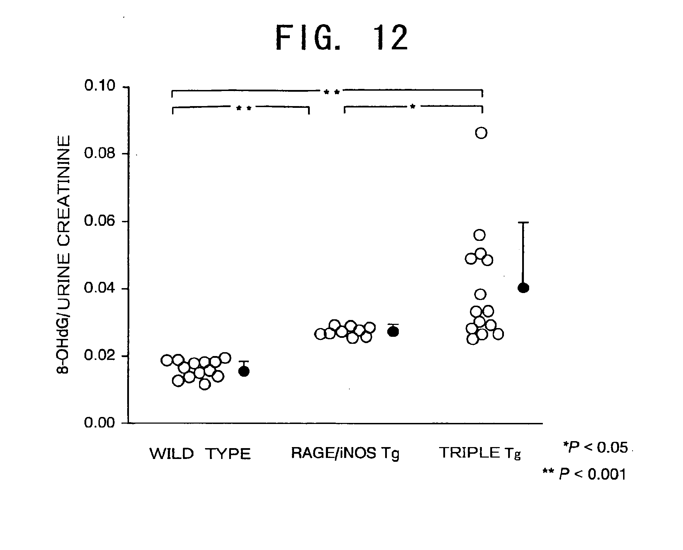

Recent studies have suggested that oxidative stress plays a pathogenic role in diabetic nephropathy. Thus, the present inventors compared the state of oxidative stress between the RAGE/iNOS-Tg and megsin/RAGE/iNOS-Tg mice. These mouse subjects were tested for their urine 8-OHdG, which is an oxidative stress marker. 8-OHdG was detected using an ELISA kit (8-OHdG Check (High sensitive); Japan Aging Control laboratories; Shizuoka, Japan). The urine used in the test was treated by ultrafiltration using Vivaspin (>10, 000 molecular weight cutoffs; Vivascience AG; Hannover, Germany), and the level of 8-OHdG in urine was then determined. The values determined were normalized using urine creatine. The result is shown in Fig. 12.

The mean urine 8-OHdG level in the megsin/RAGE/iNOS-Tg mice was two or more times higher than that in the wild type mice. This difference is statistically significant. The increase of urine 8-OHdG in the megsin/RAGE/iNOS-Tg mice was considerably higher also when compared with the RAGE/iNOS-Tg mice (Fig. 12).

### Industrial Applicability

The model animals of the present invention exhibit serious kidney function disorder (albuminuria) and glomerular pathologies (proliferation of mesangial matrix, thickening of basement membrane, and nodular sclerotic lesion) along with hyperglycemia, and in particular, exhibits clinical conditions that are extremely similar to those of human diabetic nephropathy. Conventional model animals for diabetic nephropathy exhibit only poor pathological features. In contrast, the model animals of the present invention exhibit chronic progressive pathologies at an early stage.

Specifically, glomerular hypertrophy and glomerular cell proliferation are observed in megsin/RAGE/iNOS-expressing Tg mice of the present invention at an early stage of 8 weeks or 16 weeks, and this suggests acceleration of the mesangial matrix expansion.

Such early-stage morphological abnormalities of the glomerulus result in glomerular disorder (mesangial cell activation), aggravation of tubulointerstitial disorder (fibrosis), infiltration of macrophages into the interstitium, increase in oxidative stress, and such, and 16-week-old triple Tg mice exhibit late-stage pathological conditions of diabetic nephropathy (nodular lesion, and adhesion of glomerular tuft and Bowman's capsule).

The pathological conditions of the comparative 16-week-old RAGE/iNOS-Tg remain to be early-stage pathologies (mesangial matrix expansion) and the above-described late-stage pathologies were not observed. However, the nodular lesions observed in the 16-week-old megsin/RAGE/iNOS-Tg mice are eventually detected in RAGE/iNOS-Tg at 36 weeks old.

As described above, megsin/RAGE/iNOS-Tg of the present invention develops the late-stage pathologies at an early stage, and thus the megsin/RAGE/iNOS-Tg mice are expected to contribute to the poorly advanced study of molecular pathology on late-stage pathogenesis. In addition, overexpression of megsin accelerates mesangial cell damage in the disease model animals of the present invention, and this makes it possible to compare and analyze differences in the pathophysiology of megsin and the pathophysiology of mesangial cells in diabetic nephropathy in more detail and over a wide range of lesional stages. Thus, the megsin/RAGE/iNOS-Tg mice of the present invention are useful as an evaluation model for developing novel diagnostic methods and drugs that suppress kidney disorders from advancing to late-stage pathologies.

In addition, since the incidence of the late-stage pathologies is high, the mice are useful for elucidation of the onset/progression mechanism of diabetic nephropathy and drug discovery. Furthermore, the model animals are useful for developing pharmaceuticals to treat accompanying kidney function disorders of the above-described glomerular failure. The present application provides not only the disease model animals but also a system for evaluating therapeutic agents for kidney function disorder using the model animals. Thus, the present invention is highly useful in clinical studies and development of pharmaceuticals for diseases that accompany kidney function disorders. Furthermore, the disease model animals of the present invention exhibit hyperglycemia, and thus are also expected to be highly useful in clinical studies and development of therapeutic and preventive agents for diabetes mellitus.

## Claims

1. A disease model animal expressing megsin gene, a gene encoding the receptor for advanced glycation end-products, and an inducible nitric oxide synthase gene, wherein the model animal comprises a nonhuman mammal.

2. The disease model animal of claim 1 introduced with megsin gene, a gene encoding the receptor for advanced glycation end-products, and an inducible nitric oxide synthase gene.

3. The disease model animal of claim 1 or 2, which exhibits at least one phenotype selected from the following phenotypes (a) to (f):
(a) increase in kidney-to-body weight ratio;
(b) increase in urine albumin level;
(c) increase in blood triglyceride level;
(d) underweight (hypogenesis);
(e) hyperglycemia;
(f) hypoinsulinemia; and
(g) increase in urine 8-OHdG level.

4. The disease model animal of claim 1 or 2, which exhibits in mesangial matrix at least one of the following findings:
(a) expansion of mesangial matrix;
(b) enhancement of immunoglobulin and/or complement accumulation; and
(c) increases of collagen, laminin, and/or fibronectin.

5. The disease model animal of claim 1 or 2, which exhibits in tubular interstitium the phenotypes of:
(a) fibrosis; and/or
(b) infiltration of inflammatory cells.

6. The disease model animal of any one of claims 1 to 5, wherein the megsin gene, the gene encoding the receptor for advanced glycation end-products, and the inducible nitric oxide synthase gene are derived from human.

7. The disease model animal of any one of claims 1 to 6, wherein the disease is diabetic nephropathy.

8. A method for creating a disease model animal, comprising the step of introducing megsin gene, a gene encoding the receptor for advanced glycation end-products, and an inducible nitric oxide synthase gene into a fertilized egg of a nonhuman mammal, wherein the disease model animal comprises a nonhuman mammal in which expressions of the megsin gene, the gene encoding the receptor for advanced glycation end-products, and the inducible nitric oxide synthase gene are enhanced.

9. A method for evaluating the therapeutic effect of a test compound on kidney function disorder, which comprises the steps of:
(1) administering a test compound to the disease model animal of any one of claims 1 to 7; and
(2) detecting the relieving effect on the kidney function disorder of the disease model animal administered with the test compound.

10. A method for evaluating the therapeutic effect of a test compound on kidney function disorder, which comprises the steps of:
(1) administering a test compound to the disease model animal of any one of claims 1 to 7; and
(2) measuring at least any one of kidney-to-body weight ratio, urine albumin level, blood triglyceride level, and urine 8-OHdG level in the disease model animal after administration of the test compound.

11. A method for evaluating the therapeutic effect of a test compound on kidney function disorder, which comprises the steps of:
(1) administering a test compound to the disease model animal of any one of claims 1 to 7; and
(2) determining whether the mesangial matrix of the disease model animal is altered or whether the alteration is reduced after administration of the test compound.

12. The method of claim 11, wherein the alteration of the mesangial matrix is at least one of:
(a) expansion of mesangial matrix;
(b) enhancement of immunoglobulin and/or complement accumulation; and
(c) increases of collagen, laminin, and/or fibronectin.

13. A method for evaluating the therapeutic effect of a test compound on kidney function disorder, which comprises the steps of:
(1) administering a test compound to the disease model animal of any one of claims 1 to 7; and
(2) determining whether the tubular interstitium of the disease model animal is altered or whether the alteration is reduced after administration of the test compound.

14. The method of claim 13, wherein the alteration of the tubular interstitium is:
(a) fibrosis; and/or
(b) infiltration of inflammatory cells.

15. The method of any one of claims 9 to 14, wherein the kidney function disorder is a kidney function disorder that accompanies hyperglycemia.

16. A method evaluating the therapeutic effect of a test compound on hyperglycemia, which comprises the steps of:
(1) administering a test compound to the disease model animal of any one of claims 1 to 7; and
(2) determining the glucose or/insulin level in the disease model animal after administration of the test compound.


SEQUENCE LISTING

<110> JAPAN SCIENCE AND TECHNOLOGY AGENCY
GENE THESIS CO., LTD.

<120> Model animals of diseases expressed Megsin/RAGE/iNOS triple transgenes and evaluation method
with the model animals

<130> F2-A0302P

<150> JP 2003-415779
<151> 2003-12-12

<160> 9

<170> PatentIn version 3.1

<210> 1
<211> 1143
<212> DNA
<213> Homo sapiens

<220>
<221> CDS
<222> (1)..(1140)
<223>

<400> 1
atg gcc tcc ctt gct gca gca aat gca gag ttt tgc ttc aac ctg ttc 48
Met Ala Ser Leu Ala Ala Ala Asn Ala Glu Phe Cys Phe Asn Leu Phe
1               5                   10                  15

aga gag atg gat gac aat caa gga aat gga aat gtg ttc ttt tcc tct 96
Arg Glu Met Asp Asp Asn Gln Gly Asn Gly Asn Val Phe Phe Ser Ser
            20                  25                  30

ctg agc ctc ttc gct gcc ctg gcc ctg gtc cgc ttg ggc gct caa gat 144
Leu Ser Leu Phe Ala Ala Leu Ala Leu Val Arg Leu Gly Ala Gln Asp
        35                  40                  45

gac tcc ctc tct cag att gat aag ttg ctt cat gtt aac act gcc tca 192
Asp Ser Leu Ser Gln Ile Asp Lys Leu Leu His Val Asn Thr Ala Ser
    50                  55                  60

gga tat gga aac tct tct aat agt cag tca ggg ctc cag tct caa ctg 240
Gly Tyr Gly Asn Ser Ser Asn Ser Gln Ser Gly Leu Gln Ser Gln Leu
65                  70                  75                  80

aaa aga gtt ttt tct gat ata aat gca tcc cac aag gat tat gat ctc 288
Lys Arg Val Phe Ser Asp Ile Asn Ala Ser His Lys Asp Tyr Asp Leu
                85                  90                  95

agc att gtg aat ggg ctt ttt gct gaa aaa gtg tat ggc ttt cat aag 336
Ser Ile Val Asn Gly Leu Phe Ala Glu Lys Val Tyr Gly Phe His Lys
            100                 105                 110

gac tac att gag tgt gcc gaa aaa tta tac gat gcc aaa gtg gag cga 384
Asp Tyr Ile Glu Cys Ala Glu Lys Leu Tyr Asp Ala Lys Val Glu Arg
        115                 120                 125

gtt gac ttt acg aat cat tta gaa gac act aga cgt aat att aat aag 432
Val Asp Phe Thr Asn His Leu Glu Asp Thr Arg Arg Asn Ile Asn Lys
    130                 135                 140

tgg gtt gaa aat gaa aca cat ggc aaa atc aag aac gtg att ggt gaa 480
Trp Val Glu Asn Glu Thr His Gly Lys Ile Lys Asn Val Ile Gly Glu
145                 150                 155                 160

ggt ggc ata agc tca tct gct gta atg gtg ctg gtg aat gct gtg tac 528
Gly Gly Ile Ser Ser Ser Ala Val Met Val Leu Val Asn Ala Val Tyr
                165                 170                 175

ttc aaa ggc aag tgg caa tca gcc ttc acc aag agc gaa acc ata aat 576
Phe Lys Gly Lys Trp Gln Ser Ala Phe Thr Lys Ser Glu Thr Ile Asn
            180                 185                 190

tgc cat ttc aaa tct ccc aag tgc tct ggg aag gca gtc gcc atg atg 624
Cys His Phe Lys Ser Pro Lys Cys Ser Gly Lys Ala Val Ala Met Met
        195                 200                 205

cat cag gaa cgg aag ttc aat ttg tct gtt att gag gac cca tca atg 672
His Gln Glu Arg Lys Phe Asn Leu Ser Val Ile Glu Asp Pro Ser Met
    210                 215                 220

aag att ctt gag ctc aga tac aat ggt ggc ata aac atg tac gtt ctg 720
Lys Ile Leu Glu Leu Arg Tyr Asn Gly Gly Ile Asn Met Tyr Val Leu
225                 230                 235                 240

ctg cct gag aat gac ctc tct gaa att gaa aac aaa ctg acc ttt cag 768
Leu Pro Glu Asn Asp Leu Ser Glu Ile Glu Asn Lys Leu Thr Phe Gln
                245                 250                 255

aat cta atg gaa tgg acc aat cca agg cga atg acc tct aag tat gtt 816
Asn Leu Met Glu Trp Thr Asn Pro Arg Arg Met Thr Ser Lys Tyr Val

            260                 265                 270

gag gta ttt ttt cct cag ttc aag ata gag aag aat tat gaa atg aaa 864
Glu Val Phe Phe Pro Gln Phe Lys Ile Glu Lys Asn Tyr Glu Met Lys
        275                 280                 285

caa tat ttg aga gcc cta ggg ctg aaa gat atc ttt gat gaa tcc aaa 912
Gln Tyr Leu Arg Ala Leu Gly Leu Lys Asp Ile Phe Asp Glu Ser Lys
    290                 295                 300

gca gat ctc tct ggg att gct tcg ggg ggt cgt ctg tat ata tca agg 960
Ala Asp Leu Ser Gly Ile Ala Ser Gly Gly Arg Leu Tyr Ile Ser Arg
305                 310                 315                 320

atg atg cac aaa tct tac ata gag gtc act gag gag ggc acc gag gct 1008
Met Met His Lys Ser Tyr Ile Glu Val Thr Glu Glu Gly Thr Glu Ala
                325                 330                 335

act gct gcc aca gga agt aat att gta gaa aag caa ctc cct cag tcc 1056
Thr Ala Ala Thr Gly Ser Asn Ile Val Glu Lys Gln Leu Pro Gln Ser
            340                 345                 350

acg ctg ttt aga gct gac cac cca ttc cta ttt gtt atc agg aag gat 1104
Thr Leu Phe Arg Ala Asp His Pro Phe Leu Phe Val Ile Arg Lys Asp
        355                 360                 365

gac atc atc tta ttc agt ggc aaa gtt tct tgc cct tga 1143
Asp Ile Ile Leu Phe Ser Gly Lys Val Ser Cys Pro
    370                 375                 380

<210> 2
<211> 1391
<212> DNA
<213> Homo sapiens

<220>
<221> CDS
<222> (1)..(1215)
<223>

<400> 2
ggg gca gcc gga aca gca gtt gga gcc tgg gtg ctg gtc ctc agt ctg 48
Gly Ala Ala Gly Thr Ala Val Gly Ala Trp Val Leu Val Leu Ser Leu
1               5                   10                  15

tgg ggg gca gta gta ggt gct caa aac atc aca gcc cgg att ggc gag 96

Trp Gly Ala Val Val Gly Ala Gln Asn Ile Thr Ala Arg Ile Gly Glu
            20                  25                  30

cca ctg gtg ctg aag tgt aag ggg gcc ccc aag aaa cca ccc cag cgg 144
Pro Leu Val Leu Lys Cys Lys Gly Ala Pro Lys Lys Pro Pro Gln Arg
        35                  40                  45

ctg gaa tgg aaa ctg aac aca ggc cgg aca gaa gct tgg aag gtc ctg 192
Leu Glu Trp Lys Leu Asn Thr Gly Arg Thr Glu Ala Trp Lys Val Leu
    50                  55                  60

tct ccc cag gga gga ggc ccc tgg gac agt gtg gct cgt gtc ctt ccc 240
Ser Pro Gln Gly Gly Gly Pro Trp Asp Ser Val Ala Arg Val Leu Pro
65                  70                  75                  80

aac ggc tcc ctc ttc ctt ccg gct gtc ggg atc cag gat gag ggg att 288
Asn Gly Ser Leu Phe Leu Pro Ala Val Gly Ile Gln Asp Glu Gly Ile
                85                  90                  95

ttc cgg tgc agg gca atg aac agg aat gga aag gag acc aag tcc aac 336
Phe Arg Cys Arg Ala Met Asn Arg Asn Gly Lys Glu Thr Lys Ser Asn
            100                 105                 110

tac cga gtc cgt gtc tac cag att cct ggg aag cca gaa att gta gat 384
Tyr Arg Val Arg Val Tyr Gln Ile Pro Gly Lys Pro Glu Ile Val Asp
        115                 120                 125

tct gcc tct gaa ctc acg gct ggt gtt ccc aat aag gtg ggg aca tgt 432
Ser Ala Ser Glu Leu Thr Ala Gly Val Pro Asn Lys Val Gly Thr Cys
    130                 135                 140

gtg tca gag gga agc tac cct gca ggg act ctt agc tgg cac ttg gat 480
Val Ser Glu Gly Ser Tyr Pro Ala Gly Thr Leu Ser Trp His Leu Asp
145                 150                 155                 160

ggg aag ccc ctg gtg cct aat gag aag gga gta tct gtg aag gaa cag 528
Gly Lys Pro Leu Val Pro Asn Glu Lys Gly Val Ser Val Lys Glu Gln
                165                 170                 175

acc agg aga cac cct gag aca ggg ctc ttc aca ctg cag tcg gag cta 576
Thr Arg Arg His Pro Glu Thr Gly Leu Phe Thr Leu Gln Ser Glu Leu
            180                 185                 190

atg gtg acc cca gcc cgg gga gga gat ccc cgt ccc acc ttc tcc tgt 624
Met Val Thr Pro Ala Arg Gly Gly Asp Pro Arg Pro Thr Phe Ser Cys
        195                 200                 205

agc ttc agc cca ggc ctt ccc cga cac cgg gcc ttg cgc aca gcc ccc 672
Ser Phe Ser Pro Gly Leu Pro Arg His Arg Ala Leu Arg Thr Ala Pro
    210                 215                 220

atc cag ccc cgt gtc tgg gag cct gtg cct ctg gag gag gtc caa ttg 720
Ile Gln Pro Arg Val Trp Glu Pro Val Pro Leu Glu Glu Val Gln Leu
225                 230                 235                 240

gtg gtg gag cca gaa ggt gga gca gta gct cct ggt gga acc gta acc 768
Val Val Glu Pro Glu Gly Gly Ala Val Ala Pro Gly Gly Thr Val Thr
                245                 250                 255

ctg acc tgt gaa gtc cct gcc cag ccc tct cct caa atc cac tgg atg 816
Leu Thr Cys Glu Val Pro Ala Gln Pro Ser Pro Gln Ile His Trp Met
            260                 265                 270

aag gat ggt gtg ccc ttg ccc ctt ccc ccc agc cct gtg ctg atc ctc 864
Lys Asp Gly Val Pro Leu Pro Leu Pro Pro Ser Pro Val Leu Ile Leu
        275                 280                 285

cct gag ata ggg cct cag gac cag gga acc tac agc tgt gtg gcc acc 912
Pro Glu Ile Gly Pro Gln Asp Gln Gly Thr Tyr Ser Cys Val Ala Thr
    290                 295                 300

cat tcc agc cac ggg ccc cag gaa agc cgt gct gtc agc atc agc atc 960
His Ser Ser His Gly Pro Gln Glu Ser Arg Ala Val Ser Ile Ser Ile
305                 310                 315                 320

atc gaa cca ggc gag gag ggg cca act gca ggc tct gtg gga gga tca 1008
Ile Glu Pro Gly Glu Glu Gly Pro Thr Ala Gly Ser Val Gly Gly Ser
                325                 330                 335

ggg ctg gga act cta gcc ctg gcc ctg ggg atc ctg gga ggc ctg ggg 1056
Gly Leu Gly Thr Leu Ala Leu Ala Leu Gly Ile Leu Gly Gly Leu Gly
            340                 345                 350

aca gcc gcc ctg ctc att ggg gtc atc ttg tgg caa agg cgg caa cgc 1104
Thr Ala Ala Leu Leu Ile Gly Val Ile Leu Trp Gln Arg Arg Gln Arg
        355                 360                 365

cga gga gag gag agg aag gcc cca gaa aac cag gag gaa gag gag gag 1152
Arg Gly Glu Glu Arg Lys Ala Pro Glu Asn Gln Glu Glu Glu Glu Glu
    370                 375                 380

cgt gca gaa ctg aat cag tcg gag gaa cct gag gca ggc gag agt agt 1200
Arg Ala Glu Leu Asn Gln Ser Glu Glu Pro Glu Ala Gly Glu Ser Ser
385                 390                 395                 400

act gga ggg cct tga ggggcccaca gacagatccc atccatcagc tcccttttct 1255
Thr Gly Gly Pro

ttttcccttg aactgttctg gcctcagacc aactctctcc tgtataatct ctctcctgta 1315

taaccccacc ttgccaagct ttcttctaca accagagccc cccacaatga tgattaaaca 1375

cctgacacat cttgca 1391

<210> 3
<211> 3991
<212> DNA
<213> Mus musculus

<220>
<221> CDS
<222> (185)..(3619)
<223>

<400> 3
aggccccacg ggacacagtg tcactggttt gaaacttctc agccaccttg gtgaagggac 60

tgagctgtta gagacacttc tgaggctcct cacgcttggg tcttgttcac tccacggagt 120

agcctagtca actgcaagag aacggagaac gttggatttg gagcagaagt gcaaagtctc 180

agac atg gct tgc ccc tgg aag ttt ctc ttc aaa gtc aaa tcc tac caa 229
Met Ala Cys Pro Trp Lys Phe Leu Phe Lys Val Lys Ser Tyr Gln
1               5                   10                  15

agt gac ctg aaa gag gaa aag gac att aac aac aac gtg aag aaa acc 277
Ser Asp Leu Lys Glu Glu Lys Asp Ile Asn Asn Asn Val Lys Lys Thr
                20                  25                  30

cct tgt gct gtt ctc agc cca aca ata caa gat gac cct aag agt cac 325
Pro Cys Ala Val Leu Ser Pro Thr Ile Gln Asp Asp Pro Lys Ser His
            35                  40                  45

caa aat ggc tcc ccg cag ctc ctc act ggg aca gca cag aat gtt cca 373
Gln Asn Gly Ser Pro Gln Leu Leu Thr Gly Thr Ala Gln Asn Val Pro
        50                  55                  60

gaa tcc ctg gac aag ctg cat gtg aca tcg acc cgt cca cag tat gtg 421
Glu Ser Leu Asp Lys Leu His Val Thr Ser Thr Arg Pro Gln Tyr Val

    65                  70                  75

agg atc aaa aac tgg ggc agt gga gag att ttg cat gac act ctt cac 469
Arg Ile Lys Asn Trp Gly Ser Gly Glu Ile Leu His Asp Thr Leu His
80                  85                  90                  95

cac aag gcc aca tcg gat ttc act tgc aag tcc aag tct tgc ttg ggg 517
His Lys Ala Thr Ser Asp Phe Thr Cys Lys Ser Lys Ser Cys Leu Gly
                100                 105                 110

tcc atc atg aac ccc aag agt ttg acc aga gga ccc aga gac aag cct 565
Ser Ile Met Asn Pro Lys Ser Leu Thr Arg Gly Pro Arg Asp Lys Pro
            115                 120                 125

acc cct ctg gag gag ctc ctg cct cat gcc att gag ttc atc aac cag 613
Thr Pro Leu Glu Glu Leu Leu Pro His Ala Ile Glu Phe Ile Asn Gln
        130                 135                 140

tat tat ggc tcc ttt aaa gag gca aaa ata gag gaa cat ctg gcc agg 661
Tyr Tyr Gly Ser Phe Lys Glu Ala Lys Ile Glu Glu His Leu Ala Arg
    145                 150                 155

ctg gaa gct gta aca aag gaa ata gaa aca aca gga acc tac cag ctc 709
Leu Glu Ala Val Thr Lys Glu Ile Glu Thr Thr Gly Thr Tyr Gln Leu
160                 165                 170                 175

act ctg gat gag ctc atc ttt gcc acc aag atg gcc tgg agg aat gcc 757
Thr Leu Asp Glu Leu Ile Phe Ala Thr Lys Met Ala Trp Arg Asn Ala
                180                 185                 190

cct cgc tgc atc ggc agg atc cag tgg tcc aac ctg cag gtc ttt gac 805
Pro Arg Cys Ile Gly Arg Ile Gln Trp Ser Asn Leu Gln Val Phe Asp
            195                 200                 205

gct cgg aac tgt agc aca gca cag gaa atg ttt cag cac atc tgc aga 853
Ala Arg Asn Cys Ser Thr Ala Gln Glu Met Phe Gln His Ile Cys Arg
        210                 215                 220

cac ata ctt tat gcc acc aac aat ggc aac atc agg tcg gcc atc act 901
His Ile Leu Tyr Ala Thr Asn Asn Gly Asn Ile Arg Ser Ala Ile Thr
    225                 230                 235

gtg ttc ccc cag cgg agt gac ggc aaa cat gac ttc agg ctc tgg aat 949
Val Phe Pro Gln Arg Ser Asp Gly Lys His Asp Phe Arg Leu Trp Asn
240                 245                 250                 255

tca cag ctc atc cgg tac gct ggc tac cag atg ccc gat ggc acc atc 997

Ser Gln Leu Ile Arg Tyr Ala Gly Tyr Gln Met Pro Asp Gly Thr Ile
                260                 265                 270

aga ggg gat gct gcc acc ttg gag ttc acc cag ttg tgc atc gac cta 1045
Arg Gly Asp Ala Ala Thr Leu Glu Phe Thr Gln Leu Cys Ile Asp Leu
            275                 280                 285

ggc tgg aag ccc cgc tat ggc cgc ttt gat gtg ctg cct ctg gtc ttg 1093
Gly Trp Lys Pro Arg Tyr Gly Arg Phe Asp Val Leu Pro Leu Val Leu
        290                 295                 300

caa gct gat ggt caa gat cca gag gtc ttt gaa atc cct cct gat ctt 1141
Gln Ala Asp Gly Gln Asp Pro Glu Val Phe Glu Ile Pro Pro Asp Leu
    305                 310                 315

gtg ttg gag gtg acc atg gag cat ccc aag tac gag tgg ttc cag gag 1189
Val Leu Glu Val Thr Met Glu His Pro Lys Tyr Glu Trp Phe Gln Glu
320                 325                 330                 335

ctc ggg ttg aag tgg tat gca ctg cct gcc gtg gcc aac atg cta ctg 1237
Leu Gly Leu Lys Trp Tyr Ala Leu Pro Ala Val Ala Asn Met Leu Leu
                340                 345                 350

gag gtg ggt ggc ctc gaa ttc cca gcc tgc ccc ttc aat ggt tgg tac 1285
Glu Val Gly Gly Leu Glu Phe Pro Ala Cys Pro Phe Asn Gly Trp Tyr
            355                 360                 365

atg ggc acc gag att gga gtt cga gac ttc tgt gac aca cag cgc tac 1333
Met Gly Thr Glu Ile Gly Val Arg Asp Phe Cys Asp Thr Gln Arg Tyr
        370                 375                 380

aac atc ctg gag gaa gtg ggc cga agg atg ggc ctg gag acc cac aca 1381
Asn Ile Leu Glu Glu Val Gly Arg Arg Met Gly Leu Glu Thr His Thr
    385                 390                 395

ctg gcc tcc ctc tgg aaa gac cgg gct gtc acg gag atc aat gtg gct 1429
Leu Ala Ser Leu Trp Lys Asp Arg Ala Val Thr Glu Ile Asn Val Ala
400                 405                 410                 415

gtg ctc cat agt ttc cag aag cag aat gtg acc atc atg gac cac cac 1477
Val Leu His Ser Phe Gln Lys Gln Asn Val Thr Ile Met Asp His His
                420                 425                 430

aca gcc tca gag tcc ttc atg aag cac atg cag aat gag tac cgg gcc 1525
Thr Ala Ser Glu Ser Phe Met Lys His Met Gln Asn Glu Tyr Arg Ala
            435                 440                 445

cgt gga ggc tgc ccg gca gac tgg att tgg ctg gtc cct cca gtg tct 1573
Arg Gly Gly Cys Pro Ala Asp Trp Ile Trp Leu Val Pro Pro Val Ser
        450                 455                 460

ggg agc atc acc cct gtg ttc cac cag gag atg ttg aac tat gtc cta 1621
Gly Ser Ile Thr Pro Val Phe His Gln Glu Met Leu Asn Tyr Val Leu
    465                 470                 475

tct cca ttc tac tac tac cag atc gag ccc tgg aag acc cac atc tgg 1669
Ser Pro Phe Tyr Tyr Tyr Gln Ile Glu Pro Trp Lys Thr His Ile Trp
480                 485                 490                 495

cag aat gag aag ctg agg ccc agg agg aga gag atc cga ttt aga gtc 1717
Gln Asn Glu Lys Leu Arg Pro Arg Arg Arg Glu Ile Arg Phe Arg Val
                500                 505                 510

ttg gtg aaa gtg gtg ttc ttt gct tcc atg cta atg cga aag gtc atg 1765
Leu Val Lys Val Val Phe Phe Ala Ser Met Leu Met Arg Lys Val Met
            515                 520                 525

gct tca cgg gtc aga gcc aca gtc ctc ttt gct act gag aca ggg aag 1813
Ala Ser Arg Val Arg Ala Thr Val Leu Phe Ala Thr Glu Thr Gly Lys
        530                 535                 540

tct gaa gca cta gcc agg gac ctg gcc acc ttg ttc agc tac gcc ttc 1861
Ser Glu Ala Leu Ala Arg Asp Leu Ala Thr Leu Phe Ser Tyr Ala Phe
    545                 550                 555

aac acc aag gtt gtc tgc atg gac cag tat aag gca agc acc ttg gaa 1909
Asn Thr Lys Val Val Cys Met Asp Gln Tyr Lys Ala Ser Thr Leu Glu
560                 565                 570                 575

gag gag caa cta ctg ctg gtg gtg aca agc aca ttt ggg aat gga gac 1957
Glu Glu Gln Leu Leu Leu Val Val Thr Ser Thr Phe Gly Asn Gly Asp
                580                 585                 590

tgt ccc agc aat ggg cag act ctg aag aaa tct ctg ttc atg ctt aga 2005
Cys Pro Ser Asn Gly Gln Thr Leu Lys Lys Ser Leu Phe Met Leu Arg
            595                 600                 605

gaa ctc aac cac acc ttc agg tat gct gtg ttt ggc ctt ggc tcc agc 2053
Glu Leu Asn His Thr Phe Arg Tyr Ala Val Phe Gly Leu Gly Ser Ser
        610                 615                 620

atg tac cct cag ttc tgc gcc ttt gct cat gac atc gac cag aag ctg 2101
Met Tyr Pro Gln Phe Cys Ala Phe Ala His Asp Ile Asp Gln Lys Leu
    625                 630                 635

tcc cac ctg gga gcc tct cag ctt gcc cca aca gga gaa ggg gac gaa 2149
Ser His Leu Gly Ala Ser Gln Leu Ala Pro Thr Gly Glu Gly Asp Glu
640                 645                 650                 655

ctc agt ggg cag gag gat gcc ttc cgc agc tgg gct gta caa acc ttc 2197
Leu Ser Gly Gln Glu Asp Ala Phe Arg Ser Trp Ala Val Gln Thr Phe
                660                 665                 670

cgg gca gcc tgt gag acc ttt gat gtc cga agc aaa cat cac att cag 2245
Arg Ala Ala Cys Glu Thr Phe Asp Val Arg Ser Lys His His Ile Gln
            675                 680                 685

atc ccg aaa cgc ttc act tcc aat gca aca tgg gag cca cag caa tat 2293
Ile Pro Lys Arg Phe Thr Ser Asn Ala Thr Trp Glu Pro Gln Gln Tyr
        690                 695                 700

agg ctc atc cag agc ccg gag cct tta gac ctc aac aga gcc ctc agc 2341
Arg Leu Ile Gln Ser Pro Glu Pro Leu Asp Leu Asn Arg Ala Leu Ser
    705                 710                 715

agc atc cat gca aag aac gtg ttt acc atg agg ctg aaa tcc cag cag 2389
Ser Ile His Ala Lys Asn Val Phe Thr Met Arg Leu Lys Ser Gln Gln
720                 725                 730                 735

aat ctg cag agt gaa aag tcc agc cgc acc acc ctc ctc gtt cag ctc 2437
Asn Leu Gln Ser Glu Lys Ser Ser Arg Thr Thr Leu Leu Val Gln Leu
                740                 745                 750

acc ttc gag ggc agc cga ggg ccc agc tac ctg cct ggg gaa cac ctg 2485
Thr Phe Glu Gly Ser Arg Gly Pro Ser Tyr Leu Pro Gly Glu His Leu
            755                 760                 765

ggg atc ttc cca ggc aac cag acc gcc ctg gtg cag gga atc ttg gag 2533
Gly Ile Phe Pro Gly Asn Gln Thr Ala Leu Val Gln Gly Ile Leu Glu
        770                 775                 780

cga gtt gtg gat tgt cct aca cca cac caa act gtg tgc ctg gag gtt 2581
Arg Val Val Asp Cys Pro Thr Pro His Gln Thr Val Cys Leu Glu Val
    785                 790                 795

ctg gat gag agc ggc agc tac tgg gtc aaa gac aag agg ctg ccc ccc 2629
Leu Asp Glu Ser Gly Ser Tyr Trp Val Lys Asp Lys Arg Leu Pro Pro
800                 805                 810                 815

tgc tca ctc agc caa gcc ctc acc tac ttc ctg gac att acg acc cct 2677
Cys Ser Leu Ser Gln Ala Leu Thr Tyr Phe Leu Asp Ile Thr Thr Pro

                820                 825                 830

ccc acc cag ctg cag ctc cac aag ctg gct cgc ttt gcc acg gac gag 2725
Pro Thr Gln Leu Gln Leu His Lys Leu Ala Arg Phe Ala Thr Asp Glu
            835                 840                 845

acg gat agg cag aga ttg gag gcc ttg tgt cag ccc tca gag tac aat 2773
Thr Asp Arg Gln Arg Leu Glu Ala Leu Cys Gln Pro Ser Glu Tyr Asn
        850                 855                 860

gac tgg aag ttc agc aac aac ccc acg ttc ctg gag gtg ctt gaa gag 2821
Asp Trp Lys Phe Ser Asn Asn Pro Thr Phe Leu Glu Val Leu Glu Glu
    865                 870                 875

ttc cct tcc ttg cat gtg ccc gct gcc ttc ctg ctg tcg cag ctc cct 2869
Phe Pro Ser Leu His Val Pro Ala Ala Phe Leu Leu Ser Gln Leu Pro
880                 885                 890                 895

atc ttg aag ccc cgc tac tac tcc atc agc tcc tcc cag gac cac acc 2917
Ile Leu Lys Pro Arg Tyr Tyr Ser Ile Ser Ser Ser Gln Asp His Thr
                900                 905                 910

ccc tcg gag gtt cac ctc act gtg gcc gtg gtc acc tac cgc acc cga 2965
Pro Ser Glu Val His Leu Thr Val Ala Val Val Thr Tyr Arg Thr Arg
            915                 920                 925

gat ggt cag ggt ccc ctg cac cat ggt gtc tgc agc act tgg atc agg 3013
Asp Gly Gln Gly Pro Leu His His Gly Val Cys Ser Thr Trp Ile Arg
        930                 935                 940

aac ctg aag ccc cag gac cca gtg ccc tgc ttt gtg cga agt gtc agt 3061
Asn Leu Lys Pro Gln Asp Pro Val Pro Cys Phe Val Arg Ser Val Ser
    945                 950                 955

ggc ttc cag ctc cct gag gac ccc tcc cag cct tgc atc ctc att ggg 3109
Gly Phe Gln Leu Pro Glu Asp Pro Ser Gln Pro Cys Ile Leu Ile Gly
960                 965                 970                 975

cct ggt acg ggc att gct ccc ttc cga agt ttc tgg cag cag cgg ctc 3157
Pro Gly Thr Gly Ile Ala Pro Phe Arg Ser Phe Trp Gln Gln Arg Leu
                980                 985                 990

cat gac tcc cag cac aaa ggg ctc aaa gga ggc cgc atg agc ttg gtg 3205
His Asp Ser Gln His Lys Gly Leu Lys Gly Gly Arg Met Ser Leu Val
            995                 1000                1005

ttt ggg tgc cgg cac ccg gag gag gac cac ctc tat cag gaa gaa 3250

Phe Gly Cys Arg His Pro Glu Glu Asp His Leu Tyr Gln Glu Glu
        1010                1015                1020

atg cag gag atg gtc cgc aag aga gtg ctg ttc cag gtg cac aca 3295
Met Gln Glu Met Val Arg Lys Arg Val Leu Phe Gln Val His Thr
        1025                1030                1035

ggc tac tcc cgg ctg ccc ggc aaa ccc aag gtc tac gtt cag gac 3340
Gly Tyr Ser Arg Leu Pro Gly Lys Pro Lys Val Tyr Val Gln Asp
        1040                1045                1050

atc ctg caa aag cag ctg gcc aat gag gta ctc agc gtg ctc cac 3385
Ile Leu Gln Lys Gln Leu Ala Asn Glu Val Leu Ser Val Leu His
        1055                1060                1065

ggg gag cag ggc cac ctc tac att tgc gga gat gtg cgc atg gct 3430
Gly Glu Gln Gly His Leu Tyr Ile Cys Gly Asp Val Arg Met Ala
        1070                1075                1080

cgg gat gtg gct acc aca ttg aag aag ctg gtg gcc acc aag ctg 3475
Arg Asp Val Ala Thr Thr Leu Lys Lys Leu Val Ala Thr Lys Leu
        1085                1090                1095

aac ttg agc gag gag cag gtg gaa gac tat ttc ttc cag ctc aag 3520
Asn Leu Ser Glu Glu Gln Val Glu Asp Tyr Phe Phe Gln Leu Lys
        1100                1105                1110

agc cag aaa cgt tat cat gaa gat atc ttc ggt gca gtc ttt tcc 3565
Ser Gln Lys Arg Tyr His Glu Asp Ile Phe Gly Ala Val Phe Ser
        1115                1120                1125

tat ggg gca aaa aag ggc agc gcc ttg gag gag ccc aaa gcc acg 3610
Tyr Gly Ala Lys Lys Gly Ser Ala Leu Glu Glu Pro Lys Ala Thr
        1130                1135                1140

agg ctc tga cagcccagag ttccagcttc tggcactgag taaagataat 3659
Arg Leu

ggtgaggggc ttggggagac agcgaaatgc aacccccccc aagcccctca tgtcattccc 3719

ccctcctcca ccctaccaag tagtattgta ctattgtgga ctactaaatc tctctcctct 3779

cctccctccc ctctctccct ttcctccctt cttctccact ccccagctcc ctccttctcc 3839

ttctcctcct ttgcctctca ctcttccttg gagctgagag cagagaaaaa ctcaacctcc 3899

tgactgaagc actttgggtg accaccagga ggcaccatgc cgccgctcta atacttagct 3959

gcactatgta cagatattta tacttcatat tt 3991

<210> 4
<211> 20
<212> DNA
<213> Artificial

<220>
<223> an artificially synthesized primer sequence

<400> 4
cttctggcgt gtgaccggcg 20

<210> 5
<211> 34
<212> DNA
<213> Artificial

<220>
<223> an artificially synthesized primer sequence

<400> 5
atcgaattct gagatcataa tccctgtggg atgc 34

<210> 6
<211> 17
<212> DNA
<213> Artificial

<220>
<223> an artificially synthesized primer sequence

<400> 6
agggacggag aaggagt 17

<210> 7
<211> 17
<212> DNA
<213> Artificial

<220>

<223>    an artificially synthesized primer sequence

<400>    7
tcaccccaca gactgag                                                    17


<210>    8
<211>    26
<212>    DNA
<213>    Artificial

<220>
<223>    an artificially synthesized primer sequence

<400>    8
gtgggctatg ggtttgtgga aggaga                                          26


<210>    9
<211>    20
<212>    DNA
<213>    Artificial

<220>
<223>    an artificially synthesized primer sequence

<400>    9
cgatgtcaca tgcagcttgt                                                 20